Europäisches Patentamt

European Patent Office

Office européen des brevets

(19)

(11) Veröffentlichungsnummer: **0 014 427**
**B1**

(12) **EUROPÄISCHE PATENTSCHRIFT**

(45) Veröffentlichungstag der Patentschrift:
08.05.85

(21) Anmeldenummer: 80100464.9

(22) Anmeldetag: 30.01.80

(51) Int. Cl.⁴: **A 61 L 2/10**, C 02 F 1/32 //
**A23L3/28**

(54) Mehrkammer-Photoreaktor, Mehrkammer-Bestrahlungsverfahren.

(30) Priorität: 05.02.79 DE 2904242

(43) Veröffentlichungstag der Anmeldung:
20.08.80 Patentblatt 80/17

(45) Bekanntmachung des Hinweises auf die Patenterteilung:
08.05.85 Patentblatt 85/19

(84) Benannte Vertragsstaaten:
AT BE CH DE FR GB IT LU NL SE

(56) Entgegenhaltungen:
EP - A - 0 000 773
FR - A - 2 308 409
US - A - 2 669 661
US - A - 3 637 342

Die Akte enthält technische Angaben, die nach dem
Eingang der Anmeldung eingereicht wurden und die
nicht in dieser Patentschrift enthalten sind.

(73) Patentinhaber: Schenck, Günther Otto, Prof. Dr.,
Bismarckstrasse 31, D-4330 Mülheim-Ruhr (DE)

(72) Erfinder: Schenck, Günther Otto, Prof. Dr.,
Bismarckstrasse 31, D-4330 Mülheim-Ruhr (DE)

(74) Vertreter: Wolgast, Rudolf, Dr. et al,
Bökenbusch 41 Postfach 11 03 86,
D-5620 Velbert 11 Langenberg (DE)

**Beschreibung**

Die Erfindung betrifft ein Verfahren zur Reinigung, bei dem ein fließfähiges Medium zur Einhaltung einer vorbestimmten Mindestdosis ultravioletter Strahlung im Wellenlängenbereich von 240 bis 320 nm, vorzugsweise 260 bis 280 nm, mit einem bestimmten Durchfluß durch einen Durchflußreaktor gefördert wird, der durch nicht mehr als zwei für die ultraviolette Strahlung durchlässige Trennwände senkrecht zur Durchstrahlungsrichtung in nicht mehr als drei in Durchströmungsrichtung hintereinandergeschaltete Bestrahlungskammern jeweils gleichbleibender Schichtdicke unterteilt ist, wobei ein Bruchteil von mindestens 50% der Strahlung, die in die der Strahlungsquelle unmittelbar benachbarte Bestrahlungskammer eindringt, mindestens in die direkt folgende Bestrahlungskammer fällt, und die in den Durchflußreaktor eindringende Strahlung in allen Bestrahlungskammern zusammen höchstens zu 87,5% absorbiert wird. Sie betrifft auch einen Mehrkammer-Photoreaktor zur Ausübung des Verfahrens bestehend aus einer Strahlungsquelle mit mindestens einem Strahler, aus einem durch nicht mehr als zwei für die ultraviolette Strahlung durchlässige Trennwände in nicht mehr als drei in Durchströmungsrichtung hintereinandergeschaltete Bestrahlungskammern gleichbleibender Schicht dicke unterteilten Durchflußreaktor mit einer Zuleitung und einer Ableitung für das zu bestrahlende Medium, dessen Bestrahlungskammern in bezug auf die durch die Strahlungsquelle bestimmte Durchstrahlungsrichtung hintereinander angeordnet sind, wobei die in das Medium mindestens in der Bestrahlungskammer, die der der Strahlungsquelle unmittelbar benachbarten Bestrahlungskammer direkt folgt, einfallende Strahlung einen Bruchteil von mindestens 50% der Strahlung beträgt, die in die der Strahlungsquelle unmittelbar benachbarte Bestrahlungskammer eindringt, und die Gesamtschichtdicke aller Bestrahlungskammern nach einer Absorption von nicht mehr als 87,5% der in den Durchflußreaktor eindringenden Strahlung bemessen ist.

Sowohl das erfindungsgemäße Verfahren als auch die Vorrichtung zu seiner Durchführung sind dadurch gekennzeichnet, daß das Medium durch zwei in Durchstrahlungsrichtung und in Durchströmungsrichtung unmittelbar hintereinander angeordnete Bestrahlungskammern gefördert wird, in deren engerer im Bereich von 30,8 bis 47,4% und in denen zusammen im Bereich von 64,5 bis 83,2% der eindringenden Strahlung absorbiert wird.

Besondere Ausführungsformen dieser Gegenstände sind untenstehend erläutert und in den Unteransprüchen noch im einzelnen definiert.

Bekannte Verfahren und Vorrichtungen dieser Art (EP-A-0 000 773; Nachpubl.) haben besondere Vorteile, die durch die Kombination der Wirkungen der UV-Strahlung in den verschiedenen Bestrahlungskammern gegenüber den derzeit benutzten bekannten Einkammer-Photoreaktoren, aber auch gegenüber bekannten Mehrkammer-Photoreaktoren anderer Konstruktion erzielt werden. Darin ist auch angegeben, daß eine bedeutende Zunahme in der Effizienz dadurch erreicht wird, daß die Absorption durch das Medium in der der Strahlungsquelle unmittelbar benachbarten Bestrahlungskammer auf nicht mehr als 50% und die Gesamtabsorption auf mit der Anzahl der Bestrahlungskammern zunehmende Werte, z. B. 75% bei zwei Bestrahlungskammern und 87,5% bei drei Bestrahlungskammern, begrenzt wird.

Bei einem bekannten annularen Zweikammer-Photoreaktor mit radialer Durchstrahlung (US-A-2 669 661) ist die Gesamtschichtdicke so bemessen, daß die Gesamtabsorption durch beide Bestrahlungskammern mindestens 90% beträgt, und die Trennwand zwischen den beiden Bestrahlungskammern so angeordnet, daß die auf die Trennwand fallende Strahlungsintensität der über die Apparatur gemittelten Intensität mindestens gleich ist. Das entspricht einer Absorption von nicht mehr als 53,5% in der engeren Bestrahlungskammer. Mit einer solchen Apparatur soll ein Maximum an Wirkung und Wirksamkeit erzielt werden.

Durch die FR-A-2 308 409 ist ein Photoreaktor zur Bestrahlung von Flüssigkeiten oder Gasen durch UV-Strahlung bekannt, bei dem das zu bestrahlende Medium parallel durch mehrere Bestrahlungskammern gefördert wird, von denen wenigstens zwei gleichzeitig der Strahlung von mehreren Strahlungsquellen ausgesetzt werden.

Ein weiterer bekannter Photoreaktor (US-A-3 637 342) besteht aus einem im wesentlichen zylinderförmigen Gefäß, das durch zwei UV-durchlässige Trennwände in eine mittlere Bestrahlungskammer, die von mehreren Strahlern durchsetzt ist, und zwei seitliche Bestrahlungskammern von segmentförmigem Querschnitt unterteilt ist. Die drei Bestrahlungskammern werden nacheinander von dem zu bestrahlenden Medium durchströmt.

Die vorliegende Erfindung geht von der Erkenntnis aus, daß die erzielbare Durchflußdosisleistung eines Einkammer-Photoreaktors bei einer vorgegebenen Mindestdosis durch die Schichtdicke bestimmt ist, wobei die Durchflußdosisleistung von dem Kammervolumen und von der wirksamen Strahlungsintensität abhängig ist. Die Durchflußdosisleistung $Q-M$ bei der vorbestimmten Mindestdosis M (in Milliwattsekunden pro $cm^2$) ergibt sich aus dem Durchfluß Q (in $m^3/h$) und der Strahlungsstärke (in Milliwatt pro $cm^2$) und ist eine Funktion des Volumens V des Mediums und der Strahlungsdosis E

$$Q-M = f(V, E)$$

Darin ist $Q-M$ die Durchflußdosisleistung bei der verlangten Mindestdosis M, V das Kammervolumen und E die Strahlungsintensität. Der Berechnung ist dabei die minimale in dem Kammervolumen V wirksame Strahlungsintensität und nicht die in den Photoreaktor eindringende Strahlungsintensität zugrunde zu legen. Das Kammervolumen nimmt mit zunehmender Schichtdicke d zu, während die wirksame Strahlungsintensität mit zunehmender Schichtdicke d abnimmt.

$$Q - M = V(d) \cdot E(d)$$

Es ergibt sich daraus nach den Regeln der Differentialrechnung ein Maximum der Durchflußdosisleistung

$$\frac{d(Q-M)}{d(d)} = \frac{d(V(d) \cdot E(d))}{d(d)}$$

$$0 = V(d) \cdot \frac{d(E(d))}{d(d)} + E(d) \cdot \frac{d(V(d))}{d(d)}$$

$$V(d) \cdot \frac{d(E(d))}{d(d)} = -E(d) \cdot \frac{d(V(d))}{d(d)}$$

Das Kammervolumen ist

$$V(d) = d \cdot F$$

worin F die Einstrahlungsfläche ist. Die wirksame Strahlungsintensität ist die Strahlungsintensität, die von der eindringenden Strahlung nach Durchsetzen einer Schicht des zu bestrahlenden Mediums der Schichtdicke d verbleibt.

$$E_d = E_o \cdot 10^{-\varepsilon \cdot d} \quad \text{bzw.} \quad E_d = E_o \cdot e^{-kd}$$

Für einen Einkammer-Photoreaktor mit paralleler Durchstrahlung ergibt sich daraus ein Maximum der Durchflußdosisleistung bei

$$-d \cdot F \cdot E_o \cdot k \cdot e^{-kd} = -F \cdot E_o \cdot e^{-kd}$$

d. h. $dk = 1$ bzw. $d \cdot \ln T = 1$
bzw. $\varepsilon \cdot d = \log e$ bzw. $d \cdot \log T = \log e$

Darin ist k der auf die Basis e bezogene Extinktionskoeffizient des zu bestrahlenden Mediums bei einer Wellenlänge von 254 nm, der im gesamten Wellenlängenbereich der wirksamen UV-Strahlung zugrunde gelegt wird; T ist die in einer 1-cm-Küvette, ebenfalls bei der Wellenlänge von 254 nm gemessene UV-Durchlässigkeit des zu bestrahlenden Mediums; $\varepsilon$ ist der spezifische dekadische Extinktionskoeffizient. Das Produkt $\varepsilon \cdot d$ wird üblicherweise als Extinktion bezeichnet. Unter »Absorption« wird in diesem Zusammenhang der in der Schichtdicke d absorbierte Anteil A der eindringenden Strahlungsintensität $E_o$ verstanden, der sich aus dem Lambert-Beerschen Absorptionsgesetz nach $A = E_o - E_d$ ergibt und auch in % der eindringenden Strahlungsintensität $E_o$ ausgedrückt werden kann.

Für einen Einkammer-Photoreaktor mit radial von innen nach außen gerichteter Durchstrahlung, bei dem die Bestrahlungskammer koaxial zu einem die Strahlungsquelle umgebenden Hüllrohr mit dem Radius $r_i$ angeordnet ist, sind

$$V = (r_i + d)^2 \cdot \pi \, r_i^2 = \pi \cdot d(2 r_i + d)$$

und

$$E_d = \frac{r_i}{r_i + d} \cdot E_o \cdot 10^{-\varepsilon \cdot d} \quad \text{bzw.} \quad \frac{r_i}{r_i + d} \cdot E_o \cdot e^{-kd}$$

Daraus erhält man

$$V(d) \cdot E(d) = \pi \cdot d \frac{2 r_i + d}{r_i + d} \cdot E_o \cdot e^{-kd}$$

Während die vollständige Differentiation der vorstehenden Gleichung zu einem komplizierten Ausdruck führt, ergibt eine näherungsweise Betrachtung, daß der Quotient

$$\frac{2 r_i + d}{r_i + d}$$

die Steigung der Funktion für die Durchflußdosisleistung nur wenig beeinflußt und daher näherungsweise im Rahmen der üblichen Werte für $r_i$ im Exponenten berücksichtigt werden kann. Es ist dann

$$V(d) \cdot E(d) = d \cdot F \cdot E_o \cdot e^{-Kd},$$

und das Maximum der Durchflußdosisleistung liegt bei dK = 1. Der optimierte annulare Photoreaktor und der optimierte Photoreaktor mit paralleler Durchstrahlung sind dann über $K = k \cdot \alpha$ miteinander verknüpft, und die maximale Durchflußdosisleistung des ersteren besteht bei

$$dk = \frac{1}{\alpha},$$

das empirisch zu dk = 0.826 gefunden wurde.

Die vorgenannten Einkammer-Photoreaktoren besitzen somit ein Maximum der Durchflußdosisleistung bei einer Schichtdicke d, bei der die Intensität der durch die Einstrahlungsfläche eintretenden wirksamen Strahlung $E_o$ auf $e^{-1}$, d. h. 36.8%, im Falle paralleler Durchstrahlung und auf $e^{-0.826}$, d. h. 43.7%, im Falle radialer Durchstrahlung abgefallen ist. Bei kleineren Schichtdicken verschlechtert sich die Durchflußdosisleistung wegen mangelnder Ausnutzung der zur Verfügung stehenden Strahlungsintensität. Bei höheren Schichtdicken verschlechtert sich die Durchflußdosisleistung wegen des zunehmenden Beitrags von Teilvolumina, die nur einer geringen wirksamen Strahlungsintensität ausgesetzt sind. Anders ausgedrückt ergibt sich, daß ein in seiner Schichtdicke d vorgegebener Einkammer-Photoreaktor die von der Strahlungsquelle eingestrahlte UV-Strahlung nur für Medien eines relativ eng begrenzten Bereichs von UV-Durchlässigkeiten optimal nutzt.

Die Aufgabe der vorliegenden Erfindung besteht darin, einen Mehrkammer-Photoreaktor der eingangs genannten Art zu schaffen, der eine optimale Ausnutzung der in den Reaktor eintretenden UV-Strahlung gestattet. Ein solcher Reaktor soll möglichst so beschaffen sein, daß die UV-Strahlung über weite UV-Durchlässigkeitsbereiche der zu bestrahlenden Medien, zumindest aber innerhalb der vorkommenden Variationen in der UV-Durchlässigkeit des Mediums optimal genutzt wird. Dabei soll insbesondere im Zusammenhang mit der Wasserentkeimung sichergestellt sein, daß die Strömungsgeschwindigkeit in der Strahlungsquelle unmittelbar benachbarten Bestrahlungskammer ausreicht, Abscheidungen unter der Einwirkung auch hoher Strahlungsintensitäten zu verhindern.

Diese Aufgabe wird durch die in den Patentansprüchen angegebenen kennzeichnenden Merkmale gelöst. Vorteilhafte Ausbildungen und Weiterentwicklungen der Erfindung sind durch die in den Unteransprüchen angegebenen Merkmale gekennzeichnet. Deren Besonderheiten werden im Zusammenhang mit den Ausführungsbeispielen erörtert.

Ausführungsbeispiele der erfindungsgemäßen Vorrichtung sind in den Abbildungen dargestellt und werden nachfolgend im einzelnen erläutert und beschrieben. Es zeigt

Fig. 1 eine perspektivische Ansicht eines ersten Ausführungsbeispiels des erfindungsgemäßen Mehrkammer-Photoreaktors;

Fig. 2 einen Längsschnitt durch ein zweites Ausführungsbeispiel des erfindungsgemäßen Mehrkammer-Photoreaktors;

Fig. 3 einen Längsschnitt durch ein drittes Ausführungsbeispiel des erfindungsgemäßen Mehrkammer-Photoreaktors;

Fig. 4 einen Längsschnitt durch einen Teil eines vierten Ausführungsbeispiels des erfindungsgemäßen Mehrkammer-Photoreaktors.

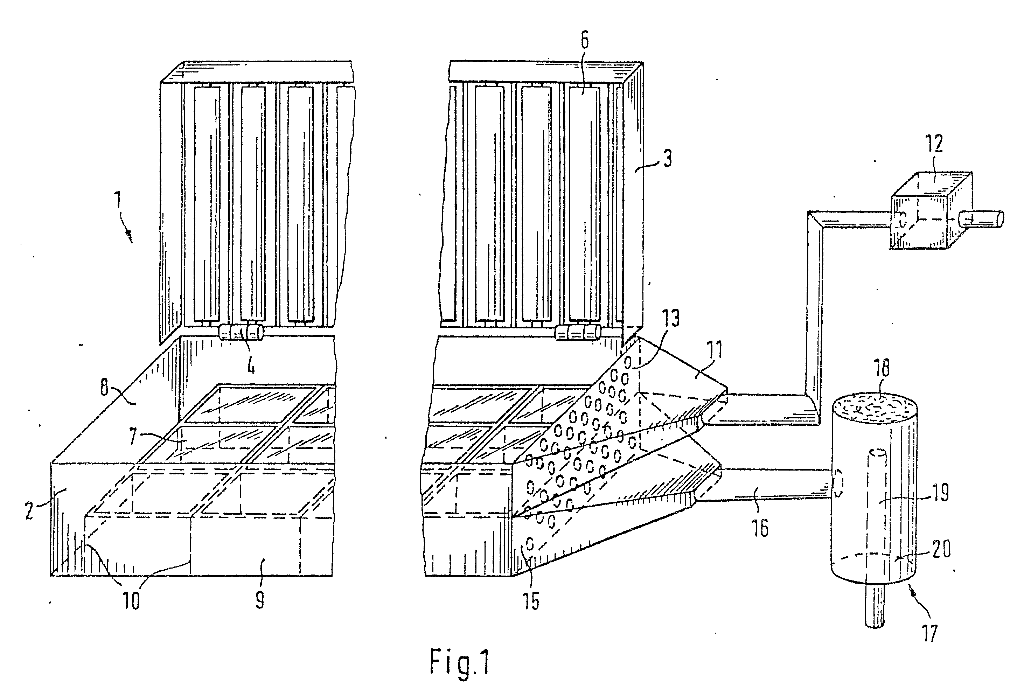

Fig. 1 zeigt einen Zweikammer-Photoreaktor 1 aus einem Durchflußreaktor in Gestalt eines trogartigen Gefäßes 2 mit einem Deckel 3, der um Scharniere 4 schwenkbar an das trogartige Gefäß 2 angelenkt ist und durch einen Schnappverschluß in geschlossener Stellung gehalten wird. Das Gefäß 2 besteht aus Metall wie rostfreiem Stahl, kann aber auch aus jedem anderen UV-beständigen und sonstigen Anforderungen, z. B. lebensmittelrechtlichen Bestimmungen, genügendem Material (Steinzeug, emailliertes Blech etc.) gefertigt sein. Der Deckel 3 trägt innen eine Serie von zueinander parallelen paraboloiden Reflektoren mit einer besonders gut UV-reflektierenden Oberfläche. Innerhalb der Reflektoren sind UV-Strahler 6 senkrecht zur Durchströmungsrichtung so angeordnet, daß der Strömungsquerschnitt des trogartigen Gefäßes 2 unter Einschluß der Randbereiche gleichmäßig bestrahlt wird. Für Zwecke der Entkeimung werden wassergekühlte, antimondotierte Xenon-Hochdrucklampen eingesetzt; alternativ eignen sich dafür auch Quecksilberniederdruck-Quarzlampen bekannter Bauart. Für die Reinigung in Anwesenheit oder Abwesenheit von Oxidationsmitteln kann man auch Quecksilberhochdrucklampen oder andere Strahler geeigneter Emissionsbereiche verwenden. Der Schnappverschluß ist mit einer Sicherheitsschaltung verbunden, durch die die Strahler 6 bei Öffnung des Schnappverschlusses automatisch abgeschaltet werden. Das trogartige Gefäß 2 ist in Strömungsrichtung durch Quarzglasscheiben 7 in zwei Bestrahlungskammern 8 und 9 unterteilt; die Bestrahlungskammer 9 ist als untere Bestrahlungskammer durch die Quarzglasscheiben 7 auf eine fixe Schichtdicke von 2 cm begrenzt, während die Schichtdicke des Mediums in der Bestrahlungskammer 8 mit Hilfe des weiter unten beschriebenen Niveaureglers 17 variiert werden kann. Die Quarzglasscheiben 7 sind auf einem herausnehmbaren Strebrahmen 10 aus rostfreiem Stahl gelagert; die Quarzglasscheiben 7 sind an dem Strebrahmen 10 und dieser selbst ist an der Innenwandung des trogartigen Gefäßes 2 mittels eines gegen UV-Strahlung beständigen Kitts abdichtend befestigt. Anstelle der Verkittung kann die Abdichtung auch durch vorgeformte und UV-beständige Dichtungen erfolgen. Die Bestrahlungskammern 8, 9 kommunizieren an ihrem dem Ein- und Ausgang des trogartigen Gefäßes 2 abgewandten Ende miteinander. Die obere Bestrahlungskammer 8 ist über eine Zuleitung 11 an einen Durchflußbegrenzer 12 angeschlossen. Der Durchflußbegrenzer dient dazu, den Durchfluß auch bei Erhöhung des Eingangsdrucks auf den zulässigen Maximalwert zu begrenzen; solche Durchflußbegrenzer werden beispielsweise von der Firma Eaton Corp., Controls Division, 191 East North Ave., Carol Stream, Illinois 60 187, USA, vertrieben. Die Zuleitung 11 mündet in die Bestrahlungskammer 8 über eine Lochplatte 13, die ein Ausgleichselement für das Strömungsprofil darstellt und sich über die gesamte Breite der Bestrahlungskammer 8 erstreckt. Die Bestrahlungskammer 9 mündet über eine gleichartige Lochplatte 15, die ebenfalls als Ausgleichselement für das Strömungsprofil wirkt, in eine Ableitung 16 mit einem Niveauregler 17, der zum Schutz gegen Verunreinigungen eine luftdurchlässige Abdeckung 18, z. B. aus Watte, trägt.

Die Lochplatten 13, 15 bestehen aus Material, das gegen UV-Strahlung und gegen das durchströmende Medium beständig ist und selbst keine störenden Verunreinigungen an das durchströmende Medium abgibt (rostfreier Stahl, beschichtete Metalle, Kunststoff, Keramik, Quarz, Glas). Die Weite der Löcher ist so groß, daß die Strömung nicht wesentlich behindert ist, aber doch ein über die Durchtrittsfläche gleichmäßiges Strömungsprofil erzeugt wird. Zu dem gleichen Zwecke können die Löcher auch durch Öffnungen anderer Gestalt wie Schlitze ersetzt werden. Die Lochplatten 13, 15 sind mit dem trogartigen Gefäß 2 einerseits und dem Übergangsstück der Zuleitung 11 bzw. der Ableitung 16 andererseits in geeigneter Weise abdichtend verkittet.

Der Niveauregler 17 besitzt ein Innenrohr 19, das abgedichtet in einem Überlaufgefäß 20 vertikal verschiebbar geführt ist und den Auslauf des trogartigen Gefäßes 2 bildet. Durch Vertikalverschiebung des Innenrohres 19 in dem Niveauregler 17 können in Anpassung an die optische Dichte des durch die Zuleitung 11 in den Durchflußreaktor eintretenden Mediums verschiedene Schichtdicken in der oberen Bestrahlungskammer 8 eingestellt werden.

Die Durchflußdosisleistung dieses Zweikammer-Photoreaktors mit paralleler Durchstrahlung setzt sich aus den Anteilen der beiden Bestrahlungskammern zusammen. Dafür gilt

$$Q-M = d_1 F \cdot E_o \cdot 10^{-\varepsilon d_1} + d_2 F \cdot E_o \cdot 10^{-\varepsilon (d_1 + d_2)}$$

Setzt man für Zwecke der Rechnung die Gesamtschichtdicke $d_1 + d_2 = D$, so ergibt sich

$$Q-M = d_1 F \cdot E_o \cdot e^{-kd_1} + (D - d_1) E_o \cdot e^{-kD}$$

Partielle Differentiation dieser Gleichung nach $d_1$ und $D$ ergibt für die maximale Durchflußdosisleistung

$$d_1 k = 1 - e^{-k(D - d_1)}$$

und

$$Dk = 1 + d_1 k$$

Daraus erhält man das Maximum der Durchflußdosisleistung bei einer Gesamtextinktion von

$$Dk = 1.632 \text{ bzw. } D\varepsilon = 0.708$$

und bei einer Extinktion in der der Strahlungsquelle 6 unmittelbar benachbarten Bestrahlungskammer 8 von

$$d_1 k = 0.632 \text{ bzw. } d_1 \cdot \varepsilon = 0.273,$$

wobei die Lichtschwächung durch die Quarzglasscheiben 7 vernachlässigt ist. Die Extinktion $d_2 \varepsilon$ der zweiten Kammer, die sich aus der Differenz der beiden vorgenannten Extinktionswerte ergibt, entspricht der Extinktion eines hinsichtlich der Durchflußdosisleistung optimierten Einkammer-Photoreaktors gleicher Bauart.

Der Zweikammer-Photoreaktor 1 weist senkrecht zur Durchströmungsrichtung 30 Quecksilberniederdruck-Quarzlampen (15 W, NN 15/44 Original Hanau Quarzlampen GmbH, Hanau) auf, die über die Bestrahlungskammern 8, 9 von 80 cm Länge in gleichen Abständen verteilt sind, wobei jeder Strahler in einem zugeordneten Reflektor und die Lampen-Reflektorkombinationen jeweils in geringstmöglichem Abstand voneinander angeordnet sind. Der gesamte auf die Oberfläche des Mediums gelangende UV-Strahlungsfluß beträgt (unter Berücksichtigung der Reflektionsverluste von höchstens 45% sowie der Randverluste) ca. 60 W mit einer mittleren Bestrahlungsstärke $E = 25$ mW/cm². Die nachfolgende Tabelle 1 zeigt die Durchflußdosisleistung $Q-M$ (m³/h) eines Zweikammer-Photoreaktors 1 mit der Gesamtschichtdicke $D = d_1 + d_2 = 4.6$ cm, wie sie sich nach der Rechnung für ein Medium mit einer UV-Durchlässigkeit von T (1 cm) = 0.7 ($\varepsilon$ = 0.155 bzw. ln T = 0.357) ergibt, in Abhängigkeit von der Schichtdicke $d_1$ für eine Mindestdosis von 40 mWs/cm$^2$. Das Maximum der Durchflußdosisleistung liegt bei $d_1$ = 1.6 bis 1.8 cm entsprechend $\varepsilon$ D = 0.248 bis 0.279. In der Tabelle sind auch die für die Praxis wichtigen UV-Durchlässigkeiten $T_1$ und $T_2$ des Mediums in den Bestrahlungskammern 8 bzw. 9 angegeben.

Tabelle 1

Durchflußdosisleistung Q−40 (m$^3$/h) eines Zweikammer-Photoreaktors mit paralleler Durchstrahlung für veränderliche Teilschichtdicken $d_1$ und $d_2$; $d_1 + d_2$ = 4.6 cm; UV-Durchlässigkeit T (1 cm) = 0.7; $T_1 \cdot T_2$ = 0.196.

| $d_1$ cm | $d_2$ cm | Q−40 m$^3$/h | $T_1$ | $T_2$ |
|---|---|---|---|---|
| 1.0 | 3.6 | 12.6 | 0.7 | 0.280 |
| 1.2 | 3.4 | 13.0 | 0.652 | 0.301 |
| 1.4 | 3.2 | 13.2 | 0.607 | 0.323 |
| 1.6 | 3.0 | 13.4 | 0.565 | 0.347 |
| 1.8 | 2.8 | 13.4 | 0.526 | 0.372 |
| 2.0 | 2.6 | 13.3 | 0.490 | 0.4 |
| 2.2 | 2.4 | 13.2 | 0.456 | 0.430 |
| 2.4 | 2.2 | 13.0 | 0.425 | 0.461 |
| 2.6 | 2.0 | 12.7 | 0.396 | 0.495 |
| 2.8 | 1.8 | 12.4 | 0.368 | 0.532 |
| 3.0 | 1.6 | 12.0 | 0.343 | 0.571 |
| 3.2 | 1.4 | 11.6 | 0.319 | 0.614 |
| 3.4 | 1.2 | 11.2 | 0.297 | 0.659 |

60 W UV − 254 nm auf 30 · 80 = 2400 cm$^2$ Einstrahlungsfläche; mittlere Bestrahlungsstärke E = 25 mW/cm$^2$.

Aus der Tabelle 1 folgt, daß sich die Durchflußdosisleistung im Bereich des Maximums nur um weniger als ±2% ändert, wenn die Schichtdicke um ±15% variiert. Das bedeutet, daß beim Bau des Zweikammer-Photoreaktors 1 keine besonderen Anforderungen an die Genauigkeit der Anordnung gestellt werden. Das bedeutet ferner, daß der Einfluß von Variationen in der UV-Durchlässigkeit des Mediums auf die Durchflußdosisleistung innerhalb gewisser Bereiche nur von geringer, unter Umständen vernachlässigbarer Bedeutung ist. So ist ein Zweikammer-Photoreaktor 1 mit einer Schichtdicke der Bestrahlungskammer 8 von $d_1$ = 1.8 cm und einer Schichtdicke der Bestrahlungskammer 9 von $d_2$ = 2.8 cm für Medien einer UV-Durchlässigkeit im Bereich von T (1 cm) 0.6 bis 0.9 einsetzbar, während ein analoger Photoreaktor mit entsprechenden Schichtdicken $d_1$ = 0.9 cm und $d_2$ = 1.4 cm für Medien mit UV-Durchlässigkeiten im Bereich von T (1 cm) 0.35 bis 0.75 verwendbar ist.

Die angegebenen Durchflußdosisleistungen Q−40 machen keine Aussage über die möglichen Durchflußleistungen, wenn nicht zugleich die kleinsten Querschnitte bzw. höchsten Strömungsgeschwindigkeiten in den Kammern berücksichtigt werden. Der vorbeschriebene Zweikammer-Photoreaktor der Tabelle 1 läßt im freien Fluß des Mediums keine hohen Strömungsgeschwindigkeiten zu. Man wird daher solche Photoreaktoren für niedrigere Durchflüsse mit höheren Mindestdosen einsetzen, kann aber auch mit Strahlungsquellen geringerer Leistung arbeiten.

Die folgende Tabelle 2 zeigt entsprechend die Durchflußdosisleistung Q−200 in m$^3$/h des Zweikam-

mer-Photoreaktors 1 für ein Medium der UV-Durchlässigkeit T (1 cm) = 0.7 bei verschiedenen Schichtdicken $d_1$ und $d_2$ und konstanter Gesamtschichtdicke $d_1 + d_2$, sowie die UV-Durchlässigkeiten der Bestrahlungskammern 8 und 9.

Tabelle 2

Durchflußdosisleistung Q−200 (m³/h) eines Zweikammer-Photoreaktors mit paralleler Durchstrahlung für veränderliche Teilschichtdicken $d_1$ und $d_2$; $d_1 + d_2 = 4.6$ cm; UV-Durchlässigkeit T (1 cm) = 0.7; $T_1 \cdot T_2 = 0.196$.

| $d_1$ cm | $d_2$ cm | Q−200 m³/h | $T_1$ | $T_2$ |
|---|---|---|---|---|
| 1.0 | 3.6 | 2.52 | 0.7 | 0.280 |
| 1.2 | 3.4 | 2.6 | 0.652 | 0.301 |
| 1.4 | 3.2 | 2.64 | 0.607 | 0.323 |
| 1.6 | 3.0 | 2.69 | 0.565 | 0.347 |
| 1.8 | 2.8 | 2.69 | 0.526 | 0.372 |
| 2.0 | 2.6 | 2.66 | 0.490 | 0.4 |
| 2.2 | 2.4 | 2.64 | 0.456 | 0.430 |
| 2.4 | 2.2 | 2.6 | 0.425 | 0.461 |
| 2.6 | 2.0 | 2.54 | 0.396 | 0.495 |
| 2.8 | 1.8 | 2.48 | 0.368 | 0.532 |
| 3.0 | 1.6 | 2.4 | 0.343 | 0.571 |
| 3.2 | 1.4 | 2.32 | 0.319 | 0.614 |
| 3.4 | 1.2 | 2.24 | 0.297 | 0.659 |

60 W UV − 254 nm auf 30 · 80 = 2400 cm² Einstrahlungsfläche; mittlere Bestrahlungsstärke E = 25 mW/cm².

Eine abgeänderte Ausführungsform des vorstehend beschriebenen Zweikammer-Photoreaktors 1 mit in parallelen Ebenen angeordneten Bestrahlungskammern besteht aus einem trogartigen Gefäß mit zwei gegenüberliegenden Wänden aus Quarzglas. Jeder dieser UV-durchlässigen Wände ist eine Strahlungsquelle zugeordnet, deren Strahler paarweise gegenüberliegend in einem System von Einzelreflektoren angeordnet sind. Durch die Überlagerung der Strahlungsfelder wird eine wesentliche Erhöhung und andere räumliche Verteilung der inneren Bestrahlungsstärke erreicht, die bei zweckmäßiger Abstimmung von Schichtdicke und Transmissionsfaktor eine Steigerung der Mindestdosisleistung von über 200% ermöglichen. So wird in einem Photoreaktor von 4.5 cm Gesamtschichtdicke bei einem Transmissionsfaktor des Mediums von T (1 cm) = 0.6 durch beidseitige Bestrahlung die dreifache Mindestdosisleistung gegenüber derjenigen erreicht, die durch die gleiche Leistung bei einseitiger Aufstrahlung erzielt werden kann.

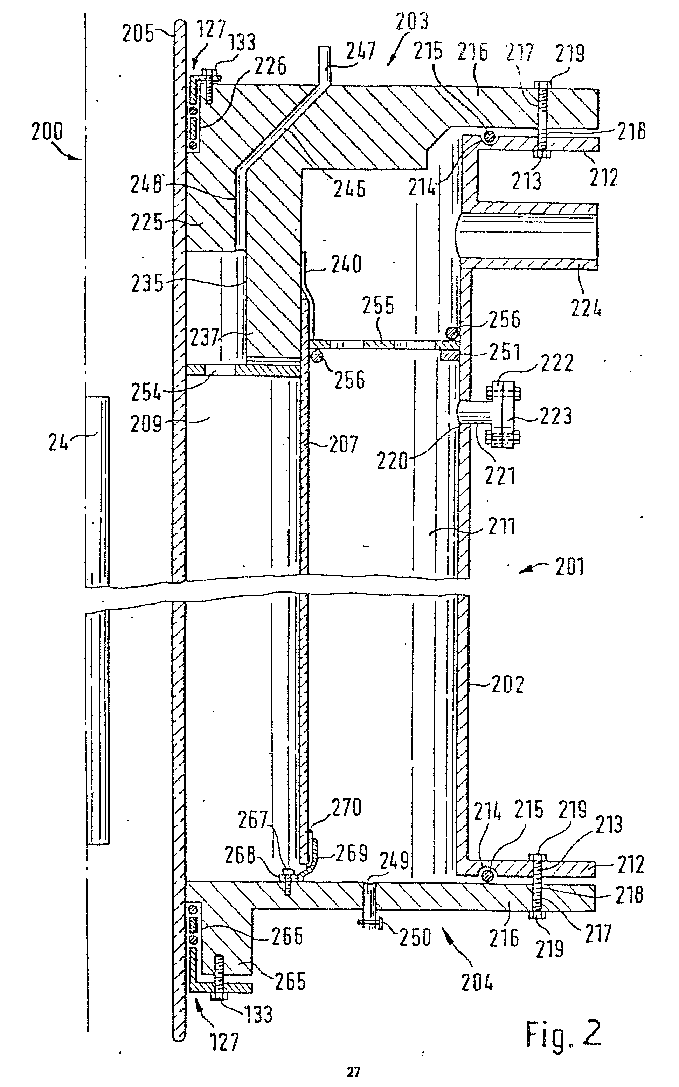

Mehrkammer-Photoreaktoren mit ringförmiger Anordnung von Strahlungsquelle und Durchflußreaktor sind aus mehreren Rohrstücken aus Quarzglas aufgebaut, die ineinander angeordnet sind und deren Durchmesser so gewählt sind, daß koaxiale Bestrahlungskammern der gewünschten Schichtdicke gebildet werden. Solche Quarzglasrohre können mit der gewünschten Genauigkeit in den Abmessungen hergestellt werden und sind mit geeigneten Durchmessern und Wandstärken im Handel erhältlich. Die Quarzglasrohre werden in bekannter Weise zueinander zentriert und zwischen Verschlußteilen (s. w. u.) gehaltert, die den Durchflußreaktor stirnseitig abschließen. Die Verschlußteile besitzen z. B. durch Stopfbuchspackungen abgedichtete Halterungsnuten für die Quarzglasrohre und sind mit Innenkanälen und Anschlußstutzen versehen, durch die die Zuleitung und Ableitung des Mediums bei Parallelschaltung und bei Sereinschaltung der Bestrahlungskammern deren Verbindung untereinander bewirkt wird. In den Abbildungen 2 bis 4 sind Ausführungsbeispiele ringförmiger Mehrkammer-Photoreaktoren mit Innenbestrahlung dargestellt.

Fig. 2 zeigt einen Zweikammer-Photoreaktor 200.

Ein Durchflußreaktor 201 ist von einem strahlungsundurchlässigen Außenmantel 202, von einem ersten Verschlußteil 203 und einem zweiten Verschlußteil 204 und von einem strahlungsdurchlässigen inneren Hüllrohr 205 gebildet, das von beiden Verschlußteilen 203 und 204 gehaltert ist. Das innere Hüllrohr 205 ist ein beidseitig offenes Quarzglasrohr. Der Durchflußreaktor 201 ist durch ein Quarzglasrohr 207, dessen Enden an den Verschlußteilen 203 bzw. 204 gehaltert sind, in zwei Bestrahlungskammern 209, 211 unterteilt.

Der Außenmantel 202 ist zur Verbindung mit den Verschlußteilen 203, 204 an beiden Enden mit Ringflanschen 212 versehen, die längs ihres Umfangs verteilte Bohrungen 213 aufweisen. An der Außenseite der Ringflansche 212 befinden sich Ausnehmungen 214 zur Aufnahme von abdichtenden O-Ringen 215. Die Verschlußteile 203, 204 tragen Flansche 216 mit längs ihres Umfangs verteilten Bohrungen 217. Der Außenmantel 202 und die Verschlußteile 203, 204 werden durch Gewindebolzen 218, die sich durch die Bohrungen 213 und 217 erstrecken und durch Muttern 219 gesichert sind, fest und abdichtend miteinander verbunden.

Der Außenmantel 202 ist wie der Außenmantel 102 des Dreikammer-Photoreaktors 100 nach Fig. 2 zu Beobachtungs- oder Kontrollzwecken mit einer Öffnung 220 und einem Tubus 221 mit Ringflansch 222 und Deckel 223 versehen. Weiterhin trägt der Außenmantel 202 nahe dem Ende, das dem Verschlußteil 203 benachbart ist, einen seitlichen Anschlußstutzen 224. Der Außenmantel 202, die Verschlußteile 203, 204 und die Quarzglasrohre 205, 207 bestehen aus dem gleichen Material wie die entsprechenden Teile des Dreikammer-Photoreaktors 100.

Die Verschlußteile 203, 204 sind allgemein ringförmig ausgebildet und haben einen Innendurchmesser, der eng an den Außendurchmesser des Hüllrohres 205 angepaßt ist. Das Verschlußteil 203 besitzt ein Axialteil 225, das sich von dem Flansch 216 her an dessen Innenseite in das Innere des Durchflußreaktors 201 erstreckt und zur Halterung des Hüllrohres 205 bzw. des Quarzglasrohres 207 an einem Ende des Durchflußreaktors 201 dient. An seinem Außenende ist das Verschlußteil 203 mit einer Gegenbohrung 226 versehen, in die eine Stopfbuchspackung 127 eingesetzt ist, die mit Schrauben 133 an der Außenfläche des Verschlußteils 203 befestigt ist und das Hüllrohr 205 an diesem Ende des Durchflußreaktors 201 fest und abdichtend haltert. An seinem inneren Ende ist das Axialteil 225 mit einer ringförmigen Ausnehmung 235 versehen, die nach außen von einem ringförmigen Steg 237 begrenzt ist. Das Axialteil 225 hat einen Außendurchmesser, der eng an den Innendurchmesser des Quarzglasrohres 207 angepaßt ist, so daß dessen eines Ende auf den Axialteil 225 aufgeschoben ist. Eine Dichtmanschette 240, die gegebenenfalls durch Ligaturen nach Art von Schlauchschellen gesichert ist, umgibt den freien Teil des Axialteils 225 und das auf den übrigen Teil aufgeschobene Ende des Quarzglasrohres 207. Dadurch wird dieses Ende des Quarzglasrohres 207 fest und abdichtend an dem Verschlußteil 203 gehaltert.

Der Verschlußteil 203 besitzt einen in seiner Außenfläche mündenden Kanal 246, der in einem Anschlußstutzen 247 endet. Der Kanal 246 ist an seinem inneren Ende mit einem Axialkanal 248 verbunden, der durch den Axialteil 225 hindurch verläuft und in den Boden der ringförmigen Ausnehmung 235 mündet. Dadurch wird eine Verbindung zwischen dem Anschlußstutzen 247 und der inneren Bestrahlungskammer 209 hergestellt.

Das Verschlußteil 204 besitzt ein Axialteil 265, das sich von dem Flansch 216 her an dessen Innenseite dem Durchflußreaktor 201 abgekehrt erstreckt und zur Halterung des Hüllrohres 205 am anderen Ende des Durchflußreaktors 201 dient. An seinem Außenende ist das Verschlußteil 204 mit einer Gegenbohrung 266 versehen, in die eine Stopfbuchspackung 127 eingesetzt ist, die mit Schrauben 133 an der Außenfläche des Verschlußteils 204 befestigt ist und das Hüllrohr 205 an diesem Ende des Durchflußreaktors 201 fest und abdichtend haltert. An seiner Innenfläche ist an dem Verschlußteil 204 mit Schrauben 267 ein Ring 268 befestigt, von dem kranzartig nach außen gewölbte Blattfedern 269 vorspringen, zwischen denen eine das andere Ende des Quarzglasrohres 207 umgebende Schutzmanschette 270 geführt ist. Das Verschlußteil 204 besitzt einen axial verlaufenden Entleerungskanal 249, der die äußere Bestrahlungskammer 211 mit einem Entleerungsventil 250 an der Außenseite des Flansches 216 verbindet.

Der Durchfluß durch den Zweikammer-Photoreaktor 200 erfolgt zwischen den Anschlußstutzen 224 und 247 durch die Bestrahlungskammern 209 und 211, die durch die (nicht dargestellten) Zwischenräume zwischen den von der Innenfläche des Verschlußteils 204 in den Durchflußreaktor 201 vorspringenden Blattfedern 269 miteinander kommunizieren. Zur Erzeugung eines gleichförmigen Strömungsprofils sind Lochplatten 254, 255 vorgesehen, die wie bei dem Dreikammer-Photoreaktor 100 ausgebildet sind. Die Lochplatte 254 ist an dem von der ringförmigen Ausnehmung 235 vorspringenden Steg 237 des Axialteils 225 vom Verschlußteil 203 befestigt und wirkt auf die die innere Bestrahlungskammer 209 durchsetzende Strömung ein. Die Lochplatte 255 liegt einem an der Innenfläche des Außenmantels 202 nahe dem Stutzen 224 befestigten Ring 251 an, der damit auch aus einem Stück gebildet sein kann; innenseitig liegt sie dem Ende der Dichtmanschette 240 an. Die Lochplatte 255 ist durch Sicherungsringe 256 gegen eine Verschiebung gesichert; sie wirkt auf die die äußere Bestrahlungskammer 211 durchsetzende Strömung ein.

Wie vorher für den Zweikammer-Photoreaktor 1 mit paralleler Durchstrahlung beschrieben, setzt sich auch die Durchflußdosisleistung des Zweikammer-Photoreaktors 200 mit radial nach außen gerichteter Durchstrahlung in entsprechender Weise aus den Durchflußdosisleistungsanteilen der beiden Bestrahlungskammern 209 und 211 zusammen. Die exakte Ableitung für das Maximum der Durchflußdosisleistung ist, wie bereits eingangs erläutert wurde, kompliziert. Daher wurde für die folgende Darstellung von der eingangs beschriebenen Näherung ausgegangen.

Ohne auf Einzelheiten einzugehen, ergibt sich auch für den Zweikammer-Photoreaktor 200, daß das Maximum der Durchflußdosisleistung innerhalb begrenzter Bereiche nur wenig von der Schichtdicke $d_1$ abhängt bzw. nur wenig von Veränderungen in der UV-Durchlässigkeit des Mediums beeinflußt wird.

Für die Anwendung, besonders auf dem Gebiet der Wasserentkeimung, ist von Bedeutung, daß die Aufgabenstellung in der Praxis unterschiedlich ist, wobei oft von einem oberen bzw. unteren Grenzwert der UV-Durchlässigkeit des Wassers ausgegangen wird. Dementsprechend zeigt die folgende Tabelle 3 für zwei Ausführungsarten des Zweikammer-Photoreaktors 200 die optimalen Werte der Gesamtschichtdicke $d_1 + d_2$ und der Schichtdicke $d_1$ der Bestrahlungskammer 209 in Abhängigkeit von der UV-Durchlässigkeit des Mediums in T (1 cm). Ausführungsart a) betrifft einen Zweikammer-Photoreaktor 200, der an Medien angepaßt ist, deren UV-Durchlässigkeit nach niedrigeren Werten veränderlich ist, während die Ausführungsart b) an Medien angepaßt ist, deren UV-Durchlässigkeit nach höheren Werten veränderlich ist.

Tabelle 3

Schichtdicken $d_1$ und $d_1 + d_2$ optimierter, angepaßter Zweikammer-Photoreaktoren 200 für Medien von UV-Durchlässigkeiten im Bereich von T (1 cm) 0.4 bis 0.9, die a) mehr nach niedrigeren, b) mehr nach höheren Werten veränderlich sind

a) $T_1 \cdot T_2 = 0.34; \quad d_1 + d_2 = \dfrac{-1.075}{\ln T (1\ cm)}; \quad d_1 = \dfrac{-0.385}{\ln T (1\ cm)}$

| $d_1 + d_2$ cm | T (1 cm) | $-\ln T (1\ cm)$ | $d_1$ cm |
|---|---|---|---|
| 10.2 | 0.9 | 0.105 | 3.654 |
| 6.615 | 0.85 | 0.163 | 2.37 |
| 4.818 | 0.8 | 0.223 | 1.725 |
| 3.737 | 0.75 | 0.288 | 1.34 |
| 3.014 | 0.7 | 0.357 | 1.08 |
| 2.495 | 0.65 | 0.431 | 0.89 |
| 2.104 | 0.6 | 0.511 | 0.75 |
| 1.173 | 0.4 | 0.916 | 0.42 |

b) $T_1 \cdot T_2 = 0.25$; $d_1 + d_2 = \dfrac{-1.376}{\ln T \,(1 \text{ cm})}$; $d_1 = \dfrac{-0.528}{\ln T \,(1 \text{ cm})}$

| $d_1 + d_2$ cm | T (1 cm) | $-\ln T$ (1 cm) | $D_1$ cm |
|---|---|---|---|
| 13.031 | 0.9 | 0.105 | 5.011 |
| 8.448 | 0.85 | 0.163 | 3.25 |
| 6.153 | 0.8 | 0.223 | 2.366 |
| 4.773 | 0.75 | 0.288 | 1.835 |
| 3.849 | 0.7 | 0.357 | 1.480 |
| 3.187 | 0.65 | 0.431 | 1.226 |
| 2.688 | 0.6 | 0.511 | 1.034 |
| 1.498 | 0.4 | 0.916 | 0.576 |
| 0.855 | 0.2 | 1.609 | 0.328 |

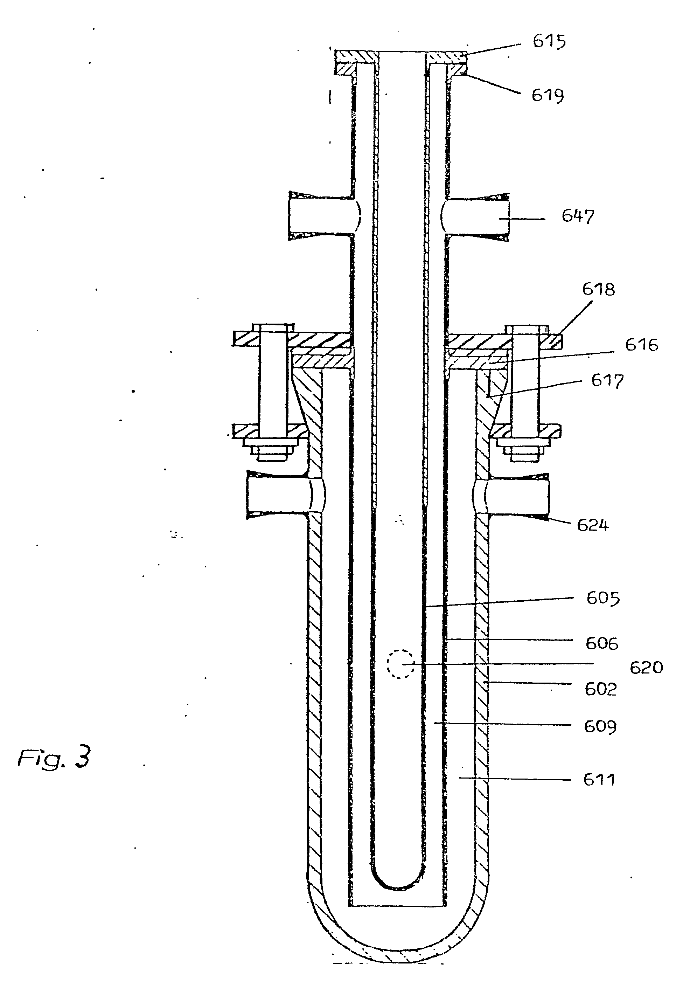

Eine weitere Ausführung eines annularen Zweikammer-Photoreaktors ist in Fig. 3 dargestellt. Der darin dargestellte Zweikammer-Photoreaktor 600 ist nach dem Tauchlampenprinzip ebenfalls koaxial aufgebaut.

Ein mit einem abgerundeten Boden versehenes, oben offenes Gefäß 602 aus Quarzgut, das auch aus Glas oder einem anderen für die UV-Strahlung undurchlässigem Material bestehen kann, ist an seinem offenen Ende mit einer plangeschliffenen Stirnfläche 612 und in diesem Bereich mit einer umfangsmäßig außen verstärkten Wandung versehen. Unterhalb des offenen Endes befinden sich zwei diametral gegenüberliegende seitliche Anschlüsse 624. Ein inneres, kurz oberhalb des abgerundeten Bodens endendes Hüllrohr 605 ist innerhalb des Gefäßes 602 geschlossen und dient zur Aufnahme des (nicht gezeigten) UV-Strahlers; im Bereich des Strahlers besteht das Hüllrohr 605 aus Quarzglas oder einem anderen für die UV-Strahlung durchlässigen Material, das mit einer strahlungsundurchlässigen Verlängerung aus Quarzgut oder einem anderen geeigneten Material versehen ist. Das Hüllrohr 605 ist von einem Trennrohr 606 aus Quarzglas oder einem anderen UV-durchlässigen Material umgeben, so daß in dem Gefäß 602 eine der Strahlungsquelle unmittelbar benachbarte Bestrahlungskammer 609 und eine weitere Bestrahlungskammer 611 gebildet werden. Im Bereich des UV-Strahlers ist das Gefäß 602 mit einer Beobachtungseinrichtung 620 versehen, die aus UV-durchlässigem Material besteht und eine in das Quarzgut-Gefäß 602 eingeschmolzene Quarzglasscheibe sein kann.

Das Trennrohr 606 ist mit einem angeschweißten Ringflansch 616 aus Quarzgut versehen. Die der Stirnfläche 612 des Gefäßes 602 zugekehrte Auflagefläche des Ringflansches ist plan geschliffen und liegt über eine Dichtung 617 aus Polytetrafluoräthylen der Stirnfläche 612 auf. Das Gefäß 602 wird mittels eines Schellenrings 618 (Schott & Gen., Mainz), der an dem Ringflansch 616 und dem verstärkten Ende des Gefäßes 602 angreift, abdichtend verschlossen. Oberhalb des Ringflansches 616 trägt das Trennrohr 606 zwei diametral gegenüberliegende seitliche Anschlüsse 647. An seinem Ende ist das Trennrohr 606 mit einem Flanschring 619 aus Quarzgut versehen, der mit einem entsprechenden Flanschring 615 am Außenende des Hüllrohres 605 verschmolzen ist, wodurch die Bestrahlungskammer 609 abdichtend verschlossen ist.

Das Hüllrohr 605 hat einen Außendurchmesser von $D_i$ = 4.6 cm bzw. einen Radius $r_i$ = 2.3 cm; das Trennrohr 606 hat einen Innendurchmesser von 7.6 cm und eine Wandstärke von 0.3 cm; das Gefäß 602 hat einen Innendurchmesser von 12.1 cm. Die Bestrahlungskammer 609 hat somit eine Schichtdicke $d_1$ = 1.5 cm und die Bestrahlungskammer 611 eine solche von $d_2$ = 2.1 cm. Der Zweikammer-Photoreaktor 600 ist unter Berücksichtigung der UV-Durchlässigkeit des Trennrohres 606 entsprechend Tabelle 3b) mehr für ein nach höheren Werten der UV-Durchlässigkeit veränderliches Medium einer UV-Durchlässigkeit von T (1 cm) = 0.7, entsprechend Tabelle 3a) mehr für ein nach niedrigeren Werten der UV-Durchlässigkeit veränderliches Medium von T (1 cm) = 0.75 ausgelegt. Tabelle 4 zeigt die Änderung der Durchflußdosisleistung Q−40 (100 Watt UV-Strahlung auf der von dem Hüllrohr 605 gebildeten Einstrahlungsfläche) mit der Änderung der Schichtdicke $d_1$ der Bestrahlungskammer 609 bei konstanter Gesamtschichtdicke $d_1 + d_2$ = 3.6 cm unter Vernachlässigung der UV-Durchlässigkeit des Trennrohres 606. Man erkennt ein breites Maximum von 12.3 m³/h bei den gewählten Schichtdicken. Die lineare Strömungsgeschwindigkeit in der Bestrahlungskammer 609 liegt im gesamten Bereich bei

# 0 014 427

günstigen Werten oberhalb 0.1 bis 0.3 m/s. In der Tabelle sind ebenfalls die bei einem Durchfluß von 5 m³/h in der Bestrahlungskammer 609 und insgesamt wirksamen Strahlungsdosen aufgetragen; das insgesamt wirksame Maximum liegt bei den gewählten Schichtdicken $d_1 = 1.5$ cm und $d_2 = 2.1$ cm.

Tabelle 4

Veränderliche Schichtdicken $d_1$, $d_2$, UV-Strahlungsdosen (Durchfluß 5 m³/h) in der Bestrahlungskammer 609, in den Bestrahlungskammern 609, 611 und hierin UV-Transmissionen $T_1$, $T_2$, Q$-$40 (m³/h) und Strömungsgeschwindigkeit $v_1$ in der Bestrahlungskammer 609 des für ein Medium mit T (1 cm) = 0.7 optimierten Zweikammer-Photoreaktors 600
100 W UV; $r_i$ = 2.3 cm; Rohr 606 Wandstärke 0.3 cm; $d_1 + d_2$ = 3.6 cm; $T_1 \cdot T_2$ = 0.354

| $d_1$ cm | $d_2$ cm | UV-Dosis für Q = 5 m³/h 2$-$K. mWs/cm² | 1$-$K. mWs/cm² | $T_1$ | $T_2$ | Q$-$40 m³/h 2$-$K. | $v_1$ m/s |
|---|---|---|---|---|---|---|---|
| 0.1 | 3.5 | 67.8 | 6.9 | 0.972 | 0.365 | 8.5 | 15.955 |
| 0.2 | 3.4 | 73.0 | 13.0 | 0.944 | 0.376 | 9.1 | 8.401 |
| 0.3 | 3.3 | 77.5 | 18.7 | 0.917 | 0.387 | 9.7 | 5.825 |
| 0.4 | 3.2 | 81.4 | 23.8 | 0.891 | 0.398 | 10.2 | 4.499 |
| 0.5 | 3.1 | 84.8 | 28.4 | 0.866 | 0.410 | 10.6 | 3.678 |
| 0.6 | 3.0 | 87.8 | 32.6 | 0.841 | 0.422 | 11.0 | 3.111 |
| 0.7 | 2.9 | 90.4 | 36.4 | 0.818 | 0.434 | 11.3 | 2.692 |
| 0.8 | 2.8 | 92.5 | 39.9 | 0.794 | 0.447 | 11.6 | 2.366 |
| 0.9 | 2.7 | 94.3 | 43.0 | 0.772 | 0.460 | 11.8 | 2.105 |
| 1.0 | 2.6 | 95.7 | 45.8 | 0.75 | 0.473 | 12.0 | 1.889 |
| 1.1 | 2.5 | 96.8 | 48.4 | 0.729 | 0.487 | 12.1 | 1.707 |
| 1.2 | 2.4 | 97.7 | 50.7 | 0.708 | 0.501 | 12.2 | 1.551 |
| 1.3 | 2.3 | 98.3 | 52.8 | 0.688 | 0.516 | 12.3 | 1.416 |
| 1.4 | 2.2 | 98.6 | 54.6 | 0.668 | 0.531 | 12.3 | 1.297 |
| 1.5 | 2.1 | 98.7 | 56.3 | 0.65 | 0.547 | 12.3 | 1.192 |
| 1.6 | 2.0 | 98.5 | 57.8 | 0.631 | 0.562 | 12.3 | 1.098 |
| 1.7 | 1.9 | 98.2 | 59.1 | 0.613 | 0.579 | 12.3 | 1.013 |
| 1.8 | 1.8 | 97.6 | 60.3 | 0.596 | 0.596 | 12.2 | 0.937 |
| 1.9 | 1.7 | 96.2 | 61.3 | 0.579 | 0.613 | 12.1 | 0.867 |
| 2.0 | 1.6 | 96.0 | 62.2 | 0.562 | 0.631 | 12.0 | 0.804 |
| 2.1 | 1.5 | 94.9 | 62.9 | 0.547 | 0.65 | 11.9 | 0.746 |
| 2.2 | 1.4 | 93.7 | 63.6 | 0.531 | 0.668 | 11.7 | 0.692 |
| 2.3 | 1.3 | 92.3 | 64.1 | 0.516 | 0.688 | 11.5 | 0.643 |
| 2.4 | 1.2 | 90.8 | 64.5 | 0.501 | 0.708 | 11.4 | 0.598 |

11

Fortsetzung

| $d_1$ cm | $d_2$ cm | UV-Dosis für $Q = 5$ m³/h 2—K. mWs/cm² | 1—K. mWs/cm² | $T_1$ | $T_2$ | $Q-40$ m³/h 2—K. | $v_1$ m/s |
|---|---|---|---|---|---|---|---|
| 2.6 | 1.0 | 87.4 | 65.1 | 0.473 | 0.75 | 10.9 | 0.516 |
| 2.8 | 0.8 | 83.5 | 65.4 | 0.447 | 0.794 | 10.4 | 0.446 |
| 3.0 | 0.6 | 79.2 | 65.3 | 0.422 | 0.841 | 9.9 | 0.384 |
| 3.2 | 0.4 | 74.5 | 65.1 | 0.398 | 0.891 | 9.3 | 0.330 |
| 3.3 | 0.3 | 72.0 | 64.9 | 0.387 | 0.917 | 9.0 | 0.305 |
| 3.4 | 0.2 | 69.4 | 64.6 | 0.376 | 0.944 | 8.7 | 0.282 |

Tabelle 5 erläutert den Anpassungsbereich des Zweikammer-Photoreaktors 600 und stellt in Abhängigkeit von der UV-Durchlässigkeit des Mediums folgendes dar:

In den Spalten 2 und 3 die bei einem Durchfluß von 5 m³/h insgesamt bzw. in der Bestrahlungskammer 609 wirksame UV-Strahlungsdosis; in Spalten 4 und 5 die UV-Transmissionen in den Bestrahlungskammern 609 bzw. insgesamt; Spalte 6 die Durchflußdosisleistung $Q-40$ in m³/h und in Spalte 7 die lineare Strömungsgeschwindigkeit in der Bestrahlungskammer 609. Wie sich aus der Tabelle ergibt, liefert der für ein Medium der UV-Durchlässigkeit von T (1 cm) = 0.7 ausgelegte Zweikammer-Photoreaktor 600 für ein Medium der UV-Durchlässigkeit von T (1 cm) = 0.5 noch eine beachtliche Durchflußdosisleistung von 5.1 m³/h bei einer Mindestdosis von 40 mWs/cm² mit der hinreichend hohen Strömungsgeschwindigkeit von $v_1$ = 0.49 m/s in der Bestrahlungskammer 609.

Tabelle 5

UV-Strahlungsdosen in der Bestrahlungskammer 609 und den Bestrahlungskammern 609, 611 und hierin UV-Transmissionen $T_1$ und $T_1 \cdot T_2$, $Q-40$ (m³/h) und Strömungsgeschwindigkeit $v_1$ in der Bestrahlungskammer 609 des für ein Medium mit T (1 cm) = 0.75 optimierten Zweikammer-Photoreaktors 600 bei verschiedenen UV-Durchlässigkeiten des Mediums 100 W UV; $r_i$ = 2.3 cm; Rohr 606 Wandstärke 0.3 cm; $d_1$ = 1.5 cm; $d_2$ = 2.1 cm

| T (1 cm) | UV-Dosis für $Q = 5$ m³/h 2—K. mWs/cm² | 1—K. mWs/cm² | $T_1$ | $T_1 \cdot T_2$ | $Q-40$ m³/h 2—K. | $v_1$ m/s |
|---|---|---|---|---|---|---|
| 1.0 | 206.0 | 86.7 | 1.0 | 1.0 | 25.8 | 2.49 |
| 0.95 | 179.5 | 80.3 | 0.926 | 0.831 | 22.4 | 2.17 |
| 0.9 | 155.7 | 74.0 | 0.854 | 0.684 | 19.5 | 1.88 |
| 0.85 | 134.4 | 67.9 | 0.784 | 0.557 | 16.8 | 1.62 |
| 0.8 | 115.5 | 62.0 | 0.716 | 0.448 | 14.4 | 1.40 |
| 0.75 | 98.7 | 56.3 | 0.65 | 0.355 | 12.3 | 1.19 |
| 0.7 | 83.8 | 50.8 | 0.586 | 0.277 | 10.5 | 1.01 |
| 0.65 | 70.7 | 45.4 | 0.524 | 0.212 | 8.8 | 0.85 |
| 0.6 | 59.3 | 40.3 | 0.465 | 0.159 | 7.4 | 0.72 |
| 0.55 | 49.2 | 35.4 | 0.408 | 0.116 | 6.2 | 0.6 |
| 0.5 | 40.5 | 30.6 | 0.354 | 0.082 | 5.1 | 0.49 |

Der Zweikammer-Photoreaktor 600 liefert nach Tabelle 5 bei Medien mit UV-Durchlässigkeiten im Bereich von $T (1 cm) = 0.5$ bis $1.0$ hohe Durchflußdosisleistungen. Insbesondere in Kombination mit UV-Strahlern mit Strahlungsleistungen $\geq 1$ W pro cm Bogenlänge (Quecksilbermitteldrucklampen mit Bogenlängen von 20 bzw. 30 cm, antimondotierte Xenonhochdrucklampen, Original Hanau Quarzlampen GmbH; Quecksilberniederdruck-Speziallampen, Fa. Gräntzel) erhält man Kompaktphotoreaktoren hoher Leistung zu vertretbaren Kosten, die bei Medien mit stark veränderlichen UV-Durchlässigkeiten eingesetzt werden können. Die hohen Strömungsgeschwindigkeiten in der der Strahlungsquelle unmittelbar benachbarten Bestrahlungskammer 609 bieten eine große Sicherheit gegen Betriebsstörungen selbst bei relativ stark veränderlicher Zusammensetzung des zu bestrahlenden Mediums. Entsprechende Anwendungsfälle sind Notwasserversorgungsanlagen, die mit Notstromaggregaten betrieben werden können. Mit den vorgenannten Hochleistungsstrahlern werden Durchflußdosisleistungen $Q-40$ im Bereich von 15 bis 20 m³/h erhalten, die für die Notwasserversorgung von 1500 Menschen ausreichen. In Entkeimungsanlagen für die Seeschiffahrt, ebenso für Fischzuchtanlagen mit Wasserkreislauf oder zu Zwecken der Muschelreinigung sind solche Kompaktreaktoren wegen ihrer Anpassung an weite UV-Durchlässigkeitsbereiche des zu bestrhalenden Wassers von besonderem Nutzen. In Verbindung mit der leistungsschwächeren Quecksilberniederdrucklampe sind die Zweikammer-Photoreaktoren 600 für die unabhängige Entkeimung von Brunnenwasser oder Quellwasser geeignet.

Andererseits kann in Kombination mit den leistungsstarken UV-Strahlern ein geringerer Durchfluß des Mediums bei sehr viel höheren Mindestdosen erhalten werden, z. B. 2 m³/h mit einer Mindestdosis von 100 mWs/cm² bei einem Medium einer UV-Durchlässigkeit von $T (1 cm) = 0.75$. So hohe Mindestdosen bei dem genannten Verbrauch werden in der pharmazeutischen und kosmetischen, aber auch in der Elektronik-Industrie verlangt.

Bei vorgegebener maximaler Strömungsgeschwindigkeit in m/s ist der maximale Durchfluß von Mehrkammer-Photoreaktoren durch den jeweils kleinsten Kammerquerschnitt bzw. bei gleichen Längen auch durch das kleinste Kammervolumen begrenzt. In der Regel dürften die Dimensionen der innersten Kammer diese begrenzende Funktion haben, da die Kammerquerschnitte bei gleichen Dikken mit zunehmendem Abstand vom Mittelpunkt bzw. von der Achse rasch zunehmen.

Ein nach Tabelle 3a) für ein Medium mit einer UV-Durchlässigkeit von $T (1 cm) = 0.9$ optimierter Zweikammer-Photoreaktor von 100 cm Länge hat die Schichtdicken $d_1 = 3.7$ cm, $d_2 = 6.5$ cm und $d_1 + d_2 = 10.2$ cm. Bei einem Hüllrohrradius $r_i = 2.3$ cm verhalten sich die in den beiden Bestrahlungskammern wirksamen Strahlungsdosen wie die Volumina bzw. Durchtrittsquerschnitte der Bestrahlungskammern. Die relativ geringe Änderung der Durchflußdosisleistung mit der Schichtdicke $d_1$ bei konstanter Gesamtschichtdicke im Bereich des Maximums ermöglicht ohne wesentliche Verschlechterung der Durchflußdosisleistung, daß die Volumina der beiden Bestrahlungskammern gleich groß gemacht werden. Dies ist bei $d_1 = 4.0$ cm und $d_2 = 6.2$ cm der Fall, wobei $Q-40$ nur vom optimalen Wert 25.91 m³/h auf 25.22 m³/h verringert wird. In der Kombination mit den vorgenannten Hochleistungsstrahlern lassen sich so die hohen Durchflußgeschwindigkeiten bei einem Durchsatz bis zu 60 m³/h wegen des geringeren Strömungswiderstandes leichter erzielen.

Im Rahmen der für den Zweikammer-Photoreaktor vorgegebenen Grenzen der Gesamtschichtdikken können also die Schichtverhältnisse den jeweiligen Erfordernissen der Strömungsgeschwindigkeiten bei hohen oder niedrigen Durchflüssen weitgehend angepaßt werden, wobei die Durchflußdosisleistung stets wesentlich oberhalb der mit Einkammer-Photoreaktoren erreichbaren liegt.

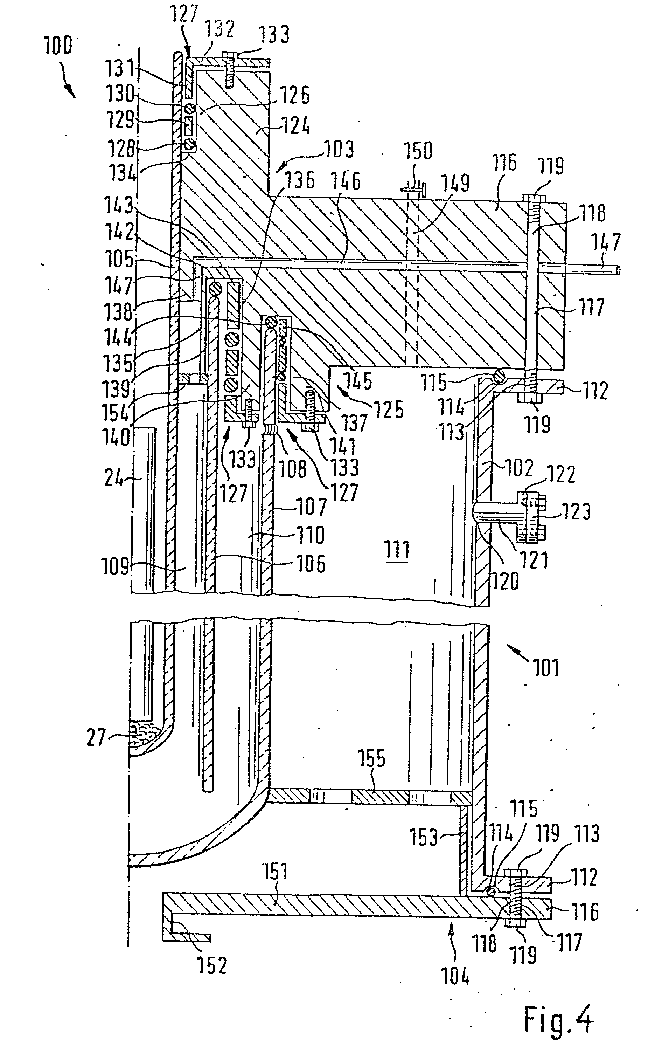

Ein für Innenbestrahlung eingerichteter Dreikammer-Photoreaktor 100 ist in Fig. 4 zur Hälfte im Längsschnitt dargestellt. Er enthält einen Strahler 24 der vorgenannten Art, der zur Erhöhung der Bestrahlungsstärke im Photoreaktor 100 einfach oder mehrfach gewendelt sein kann. Der Strahler 24 ist achsnah im Inneren eines Durchflußreaktors 101 angeordnet, der von einem strahlungsundurchlässigen Außenmantel 102, von einem ersten Verschlußteil 103 und einem zweiten Verschlußteil 104 und von einem strahlungsundurchlässigen inneren Hüllrohr 105, das in dem ersten Verschlußteil 103 gehaltert ist, gebildet ist. Das innere Hüllrohr 105 ist ein einseitig geschlossenes Quarzglasrohr, an dessen geschlossenem Ende der Strahler 24 auf einer Glaswollepackung 27 aufliegt. Der Durchflußreaktor 101 ist durch ein Quarzglasrohr 106 und ein einseitig geschlossenes Quarzglasrohr 107 mit Durchtrittsöffnungen 108 in der Wand an seinem offenen Ende, die beide ebenfalls in dem ersten Verschlußteil 103 gehaltert sind, in drei Bestrahlungskammern 109, 110, 111 unterteilt.

Der Außenmantel 102 ist zur Verbindung mit den Verschlußteilen 103, 104 an beiden Enden mit Ringflanschen 112 versehen, die längs ihres Umfangs verteilte Bohrungen 113 aufweisen. An der Außenseite der Ringflansche 112 befinden sich Ausnehmungen 114 zur Aufnahme von abdichtenden O-Ringen 115. Die Verschlußteile 103 und 104 tragen Flansche 116 mit längs ihres Umfangs verteilten Bohrungen 117, deren Zahl und Durchmesser den Bohrungen 113 in den Ringflanschen 112 des Außenmantels 102 entsprechen. Der Außenmantel 102 und die Verschlußteile 103, 104 werden mit den Ringflanschen 112 und den Flanschen 116 so angeordnet, daß die Bohrungen 113 und 117 fluchten, so daß diese Teile durch Gewindebolzen 118, die sich durch die Bohrungen 113 und 117 erstrecken, und Muttern 119 fest miteinander verbunden werden können.

Der Außenmantel 102 ist zu Beobachtungs- oder Kontrollzwecken im Bereich des Strahlungsfeldes des Strahlers 24 mit einer Öffnung 120 versehen, in die ein Tubus 121 mit einem äußeren Ringflansch 122 eingepaßt ist. Bei Nichtgebrauch ist der Tubus 121 durch einen fest und dicht, z. B. durch Verschrauben, mit dem Ringflansch 122 verbundenen Deckel 123 verschlossen. Im Gebrauch ist der Tubus 121 über ein Quarzfenster mit dem Photoreaktor einer Überwachungseinrichtung für die durch den Durchflußreaktor 101 hindurchtretende Strahlung verbunden. Der Außenmantel 102 kann zwecks Ausnutzung der bei hohem Transmissionsfaktor des Mediums auf den Außenmantel 102 aufgestrahlten UV-Leistung mit einem die UV-Strahlen in das Medium reflektierenden Material versehen werden. Bei Verwendung eines Außenmantels aus Quarz läßt sich die reflektierende Oberfläche auch auf der Außenseite anordnen, wodurch Beeinflussungen des Reflektionsvermögens durch das Medium vermieden werden.

Der Außenmantel 102 und die Verschlußteile 103, 104 bestehen aus Metall wie rostfreiem Stahl, aus Metallen mit einem Schutzüberzug aus Glas, Emaille oder Kunststoff, aus verzinktem Eisenblech, aus Keramik; es kann dafür jedes Material geeigneter mechanischer Festigkeit Verwendung finden, das beständig gegen UV-Strahlung ist und keine Fremdstoffe oder Schadstoffe an das durchfließende Medium abgibt. Zur Erhöhung der mechanischen Festigkeit und zur Erleichterung der Verarbeitung und Handhabung können das Hüllrohr 105 und die Quarzglasrohre 106, 107 in den Bereichen, die außerhalb des Strahlungsfeldes des Strahlers 24 liegen, mit Verlängerungsstücken, z. B. aus Sinterquarz, verschmolzen sein.

Das Verschlußteil 103 ist allgemein ringförmig ausgebildet und hat einen Innendurchmesser, der eng an den Außendurchmesser des Hüllrohres 105 angepaßt ist. Das ringförmige Verschlußteil 103 trägt zwei Axialteile 124, 125, die sich zu beiden Seiten des Flansches 116 an dessen Innenrand erstrecken und zur Halterung des Hüllrohres 105 bzw. der Quarzglasrohre 106 und 107 dienen. Das erste Axialteil 124 ist an seinem Außenende mit einer Gegenbohrung 126 versehen, in die eine Stopfbuchspackung 127 eingesetzt ist. Die Stopfbuchspackung 127 besteht aus zwei durch einen Führungsring 129 getrennten O-Ringen 128, 130, die durch einen Anpreßring 131 mit einem Ringflansch 132, der durch Schrauben 133 an der Außenfläche des ersten Axialteils 124 befestigt ist, gegen die am Ende der Gegenbohrung 126 ausgebildete Schulter 134 gedrückt werden. Dadurch wird das Hüllrohr 105 fest und abgedichtet an dem ersten Axialteil 124 gehaltert. Das zweite Axialteil 125 ist von innen her mit drei konzentrischen Ringnuten 135, 136 und 137 versehen, deren Tiefe von innen nach außen abnimmt und die ringförmige Stege 138, 139, 140 und 141 ausbilden. Die Stege 138 und 139 besitzen geringe und unterschiedliche axiale Tiefe und begrenzen die innerste, tiefste Ringnut 135. Die mittlere Ringnut 136 wird von dem Steg 139 und dem längeren Steg 140 begrenzt, während die äußerste, flachste Ringnut 137 von zwei gleich tiefen Stegen 140, 141 eingeschlossen ist. Die mittlere Ringnut 136 dient zur Aufnahme des Quarzglasrohres 106, dessen Ende über einen O-Ring 142 dem Boden der Ringnut 136 anliegt; eine Buchse 143 umschließt den O-Ring 142 und das obere Ende des Quarzglasrohres 106. Das Quarzglasrohr 106 wird durch eine Stopfbuchspackung 127, die mit Schrauben 133 an der Außenfläche des Stegs 140 befestigt ist, fest und abgedichtet in der mittleren Ringnut 136 gehalten. Die äußere Ringnut 137 dient zur Aufnahme des einseitig geschlossenen Quarzglasrohres 107, dessen offenes Ende über einen O-Ring 144 dem Boden der Ringnut 137 anliegt; eine Buchse 145 umschließt den O-Ring 144 und das offene Ende des einseitig geschlossenen Quarzglasrohres 107. Das Quarzglasrohr 107 wird durch eine Stopfbuchspackung 127, die mit Schrauben 133 an der Außenfläche des Stegs 141 befestigt ist, fest und abgedichtet oberhalb der Durchtrittsöffnungen 108 in der äußeren Ringnut 137 gehaltert.

Das Verschlußteil 103 besitzt zwei diametral gegenüber in der Umfangsfläche des Flansches 116 mündende Radialkanäle 146, die in Anschlußstutzen 147 enden. An ihrem inneren Ende sind die Radialkanäle 146 mit einem rechtwinklig abzweigenden Axialkanal 148 verbunden, der in den Boden der Ringnut 135 mündet. Dadurch wird eine Verbindung zwischen dem Anschlußstutzen 147 und der inneren Bestrahlungskammer 109 hergestellt. Der Flansch 116 besitzt zusätzlich einen axial verlaufenden Entlüftungskanal 149, der die äußere Bestrahlungskammer 111 mit einem Entlüftungsventil 150 an der Außenseite des Flansches 116 verbindet.

Das Verschlußteil 104 besteht aus einer Platte 151 mit einem zentralen Anschlußstutzen 152. Der Innenfläche der Platte 151 liegt ein Ring 153 auf, der umfangsmäßig der Innenwandung des Außenmantels 102 anliegt.

Der Durchfluß durch den Dreikammer-Photoreaktor 100 erfolgt zwischen den Anschlußstutzen 147 und 152 durch die Bestrahlungskammern 109, 110 und 111, wobei die Bestrahlungskammern 110 und 111 durch die Durchtrittsöffnungen 108 in der Wand des einseitig geschlossenen Quarzglasrohres 107 miteinander kommunizieren. Zur Erzeugung eines gleichförmigen Strömungsprofils sind ringförmige Lochplatten 154, 155 vorgesehen. Die Lochplatte 154 ist an dem Steg 139 des zweiten Axialteils 125 des ersten Verschlußteils 103 befestigt und wirkt auf die die innere Bestrahlungskammer 109 durchsetzende Strömung ein. Die Lochplatte 155 liegt dem der Innenfläche der Platte 151 des zweiten Verschlußteils 104 aufliegenden Ring 153 an und wirkt auf die die äußere Bestrahlungskammer 111 durchsetzende Strömung ein; an ihrer Innenkante liegt das Quarzglasrohr 107 an, das dadurch an seinem geschlossenen Ende zusätzlich geführt ist. Die Lochplatten 154, 155 bestehen aus Material, das gegen UV-Strahlung und gegen das durchströmende Medium beständig ist und selbst keine Fremd- oder Schadstoffe an das Medium abgibt (rostfreier Stahl, beschichtete Metalle, Kunststoff, Keramik, Quarz, Glas). Die Weite der Löcher ist so groß, daß die Strömung nicht wesentlich behindert ist, jedoch ein über die Durchtrittsfläche gleichmäßiges Strömungsprofil erzeugt wird. Dazu können die Löcher auch durch Öffnungen anderer geeigneter Gestalt wie Schlitze ersetzt werden.

Für den Dauerbetrieb des Dreikammer-Photoreaktors 100 spielt die Durchströmungsrichtung kaum eine Rolle. Wesentliche Unterschiede können sich jedoch beim Anlaufen des Betriebes ergeben. Bei wiederholten Unterbrechungen im Betrieb kann es erwünscht sein, schon nach kürzester Anlaufzeit Medium des geforderten Reinheits- bzw. Entkeimungsgrades zu erhalten. Dann ist es zweckmäßig, das Medium über den Anschlußstutzen 152 von der äußeren Bestrahlungskammer 111 durch die innere Bestrahlungskammer 109 zum Anschlußstutzen 147 strömen zu lassen. Mit der gleichen Durchströmungsrichtung kann in Fällen von Niederschlagsbildung erreicht werden, daß der störende Effekt zunächst auf die äußeren Bestrahlungskammern beschränkt bleibt und nicht zu schnell das Gesamtergebnis in Frage stellt. Aus Gründen der Lampenkühlung wird man im allgemeinen jedoch die Strömungsrichtung von innen nach außen bevorzugen, ebenso in Fällen der Begasung.

Der in Fig. 4 dargestellte Dreikammer-Photoreaktor 100 stellt eine Kombination eines hinsichtlich der Durchflußdosisleistung optimierten Zweikammer-Photoreaktors dar, der mit einer zusätzlichen, der Strahlungsquelle unmittelbar benachbarten Bestrahlungskammer 109 in der Weise kombiniert ist, daß eine optimale Durchflußdosisleistung erzielt wird. Tabelle 6 zeigt die Daten für solche Kombinationen, die in Tabelle 6a) und 6b) entsprechend Tabelle 3a) bzw. 3b) an Medien mit nach niedrigeren bzw. höheren Werten veränderlichen UV-Durchlässigkeiten angepaßt sind.

Tabelle 6

Schichtdicken $d_1$, $d_2$, $d_2 + d_3$ optimierter, angepaßter Dreikammer-Photoreaktoren 100 für Medien von UV-Durchlässigkeiten im Bereich von T (1 cm) 0.2 bis 0.9, die a) nach niedrigeren, b) nach höheren Werten veränderlich sind.

a) $T_2 \cdot T_3 = 0.34$

$$d_2 + d_3 = \frac{-1.075}{\ln T (1\ cm)}\ cm, \quad d_2 = \frac{-0.385}{\ln T (1\ cm)}\ cm, \quad d_1 = \frac{-0.328}{\ln (T (1\ cm)}\ cm$$

| $d_2 + d_3$ cm | T (1 cm) | $-\ln T$ (1 cm) | $d_2$ cm | $d_1$ cm |
|---|---|---|---|---|
| 10.2 | 0.9 | 0.105 | 3.654 | 3.113 |
| 6.615 | 0.85 | 0.163 | 2.37 | 2.02 |
| 5.588 | 0.825 | 0.192 | 2.001 | 1.705 |
| 4.818 | 0.8 | 0.223 | 1.725 | 1.47 |
| 3.737 | 0.75 | 0.288 | 1.34 | 1.14 |
| 3.014 | 0.7 | 0.357 | 1.08 | 0.92 |
| 2.495 | 0.65 | 0.431 | 0.89 | 0.761 |
| 2.104 | 0.6 | 0.511 | 0.75 | 0.642 |
| 1.173 | 0.4 | 0.916 | 0.42 | 0.358 |

b) $T_2 \cdot T_3 = 0.25$

$$d_2 + d_3 = \frac{-1.376}{\ln T (1\ cm)}\ cm, \quad d_2 = \frac{-0.528}{\ln T (1\ cm)}\ cm, \quad d_1 = \frac{-0.446}{\ln T (1\ cm)}\ cm$$

| $d_2 + d_3$ cm | T (1 cm) | $-\ln T$ (1 cm) | $d_2$ cm | $d_1$ cm |
|---|---|---|---|---|
| 13.031 | 0.9 | 0.105 | 5.011 | 4.233 |
| 8.448 | 0.85 | 0.163 | 3.25 | 2.744 |
| 6.153 | 0.8 | 0.223 | 2.366 | 1.999 |
| 4.773 | 0.75 | 0.288 | 1.835 | 1.550 |
| 3.849 | 0.7 | 0.357 | 1.480 | 1.25 |
| 3.187 | 0.65 | 0.431 | 1.226 | 1.035 |
| 2.688 | 0.6 | 0.511 | 1.034 | 0.873 |
| 1.498 | 0.4 | 0.916 | 0.576 | 0.487 |
| 0.855 | 0.2 | 1.609 | 0.328 | 0.277 |

Der Dreikammer-Photoreaktor kann aber auch in umgekehrter Reihenfolge so aufgebaut sein, daß ein hinsichtlich der Durchflußdosisleistung für Medien einer UV-Durchlässigkeit im Bereich von T (1 cm) 0.4 bis 0.6 optimierter Zweikammer-Photoreaktor mit einer in Strahlungsrichtung nachgeschalteten Bestrahlungskammer 111 größerer Schichtdicke ($d_3 = 3.5$ cm) kombiniert wird. Die Tabelle 7 zeigt für Medien von UV-Durchlässigkeiten im Bereich von T (1 cm) 0.2 bis 0.95 die in den Bestrahlungskammern 109, 110 und 111 wirksamen UV-Strahlungsdosen, sowie die UV-Durchlässigkeit bzw. die Durchflußdosisleistung Q−40 insgesamt und die der den Zweikammer-Photoreaktor bildenden inneren Bestrahlungskammern 109 und 110. Man erkennt, daß dieser Dreikammer-Photoreaktor nahezu universal im Bereich der vorkommenden UV-Durchlässigkeiten für Wasser einsetzbar ist. So kommt im Bereich niedriger UV-Durchlässigkeiten des Mediums (unterhalb T (1 cm) =0.6) praktisch nur der aus den Bestrahlungskammern 109 und 110 gebildete Zweikammer-Photoreaktor zur Wirkung, während bei höheren UV-Durchlässigkeiten des Mediums (oberhalb T (1 cm) = 0.6) der in der Durchflußdosisleistung erheblich günstigere Dreikammer-Photoreaktor immer stärker wirksam wird. Eine so weitgehende Anpassung eines Photoreaktors an derart unterschiedliche UV-Durchlässigkeiten läßt sich bei Zweikammer-Photoreaktoren nicht erzielen.

Tabelle 7

Universal-Dreikammer-Photoreaktor: Durchflußdosisleistung Q−40 und UV-Dosisverteilung bei konstantem Durchfluß Q = 5 m³/h eines annularen Universal-Dreikammer-Photoreaktors.
$r_i = 2.3$ cm; $d_1 = 0.75$; $d_2 = 1.1$; $d_3 = 3.5$ cm; $v_1 (d_1) = 4.538$ m/s.
100 W radiale Einstrahlung in das Medium.

| T (1 cm) | UV-Dosisverteilung für Q = 5 m³/h | | | 3−K. $T_{ges.}$ $T_1 \times T_2 \times T_3$ | 2−K. $T_{ges.}$ $T_1 \times T_2$ | Q−40 m³/h | |
|---|---|---|---|---|---|---|---|
| | 3−K. mWs/cm² | 2−K. (1+2) mWs/cm² | 1−K. (1) mWs/cm² | | | 3−K. | 2−K. |
| 0.95 | 236.8 | 100.8 | 45.6 | 0.764 | 0.914 | 29.6 | 12.6 |
| 0.9 | 196.4 | 94.0 | 43.8 | 0.575 | 0.832 | 24.6 | 11.8 |
| 0.85 | 163.2 | 87.4 | 41.9 | 0.426 | 0.752 | 20.4 | 10.9 |
| 0.8 | 136.1 | 81.0 | 40.1 | 0.310 | 0.677 | 17.0 | 10.1 |
| 0.75 | 114.0 | 74.7 | 38.2 | 0.221 | 0.604 | 14.3 | 9.3 |

Fortsetzung

| T (1 cm) | UV-Dosisverteilung für Q = 5 m³/h | | | 3—K. T$_{ges.}$ | 2—K. T$_{ges.}$ | Q—40 m³/h | |
| | 3—K. mWs/cm² | 2—K. (1+2) mWs/cm² | 1—K. (1) mWs/cm² | T$_1$ × T$_2$ × T$_3$ | T$_1$ × T$_2$ | 3—K. | 2—K. |
| --- | --- | --- | --- | --- | --- | --- | --- |
| 0.7 | 96.0 | 68.6 | 36.2 | 0.154 | 0.536 | 12.0 | 8.6 |
| 0.65 | 81.3 | 62.7 | 34.3 | 0.104 | 0.471 | 10.2 | 7.8 |
| 0.6 | 69.2 | 57.0 | 32.3 | 0.068 | 0.409 | 8.65 | 7.13 |
| 0.55 | 59.2 | 51.5 | 30.2 | 0.043 | 0.351 | 7.4 | 6.4 |
| 0.5 | 50.8 | 46.1 | 28.2 | 0.0263 | 0.297 | 6.4 | 5.8 |
| 0.45 | 43.7 | 41.0 | 26.0 | 0.0151 | 0.247 | 5.5 | 5.1 |
| 0.4 | 37.4 | 36.0 | 23.8 | 0.0081 | 0.201 | 4.7 | 4.5 |
| 0.35 | 31.9 | 31.2 | 21.6 | 0.0040 | 0.159 | 4.0 | 3.9 |
| 0.3 | 26.9 | 26.5 | 19.2 | 0.0018 | 0.122 | 3.4 | 3.3 |
| 0.25 | 22.2 | 22.1 | 16.7 | 0.0007 | 0.088 | 2.8 | 2.8 |
| 0.2 | 17.8 | 17.8 | 14.2 | 0.0002 | 0.06 | 2.2 | 2.2 |

Vorstehend sind zur grundsätzlichen Klärung der Zusammenhänge die Verhältnisse an Ausführungsbeispielen mit UV-Strahlern relativ großer Länge dargestellt worden. In der Praxis muß jedoch die endliche Länge der UV-Strahler berücksichtigt werden. Deren Abstrahlung erfolgt nicht punktförmig und auch nicht von einer Linie von Punktquellen, sondern von unterschiedlichen mit Punktquellen besetzten Flächen. Wegen der nach verschiedenen Winkeln erfolgenden Ab- und Einstrahlung bildet sich im Medium ein Bestrahlungsstärkeprofil aus, das sich durch verstärkte Absorption in Strahlernähe auszeichnet, weshalb die wirkliche Bestrahlungsstärke mit zunehmendem Abstand vermehrt unterhalb der berechneten Bestrahlungsstärke liegt und dieser Effekt sich mit abnehmender UV-Durchlässigkeit des Mediums verstärkt. Bei der Dimensionierung der Mehrkammer-Photoreaktoren sind noch Korrekturen für nicht ideale Strahlungsquellen erforderlich, um den Reaktor an das reale Bestrahlungsstärkeprofil im Medium anzupassen, wobei die Unterteilung des Reaktors und die Verkürzung der Schichten d$_1$ sich vorteilhaft auswirken (S. M. Jacob, J. S. Dranoff, AlChE Journal, Vol. 16, Nr. 3 [1970], S. 359 bis 363).

Der vorstehend im Zusammenhang mit Fig. 4 und Tabelle 7 beschriebene Dreikammer-Photoreaktor zeigt einen der Vorzüge des Mehrkammerprinzips, nämlich die optimale Anpassung eines Photoreaktors an einen sehr weiten Bereich unterschiedlicher Wasserqualitäten. Das Mehrkammerprinzip bietet aber auch besondere Vorteile in einem Bereich von Wasserqualitäten für höchste Ansprüche, wie sie z. B. in der Elektronik-Industrie, in der pharmazeutischen Industrie oder Medizin z. B. für Aqua ad injectabilia gestellt werden. Die hier im allgemeinen durch Destillation, Deionisierung, Ultrafiltration, Umkehrosmose, Filtration und Adsorption bereitgestellten Wasserqualitäten übersteigen mit ihren UV-Durchlässigkeiten im allgemeinen eine Transmission T (1 cm) = 0.9 erheblich und sollten im Endprodukt oberhalb T (1 cm) = 0.98 liegen. Für den Transmissionsbereich um T (1 cm) = 0.9 optimierte Mehrkammer-Photoreaktoren sind auch für diese höchsten Wasserqualitäten geeignet, obwohl sie nicht dafür gemäß den angegebenen Formeln optimiert sind, jedoch würde die Optimierung nach den Reinstwasserqualitäten zu technisch schwierigen und aufwendigen Konstruktionen führen, bei denen sich darüber hinaus auch die Strömungsverhältnisse verschlechtern würden. Eine Anpassung des Photoreaktors an eine während der Bestrahlung zunehmende UV-Durchlässigkeit des Wassers ist besonders dann erwünscht, wenn außer der bakteriziden Wirkung noch eine oxidative Entfernung von organischen Verunreinigungsspuren angestrebt wird. Überraschenderweise wurde hier nun gefunden, daß ein für eine UV-Durchlässigkeit von T (1 cm) = 0.9 optimierter Dreikammer-Photoreaktor auch bei UV-Durchlässigkeiten des Reinstwasserbereichs im Vergleich zu den heute überwiegend verwendeten Einkammer-Photoreaktoren in der Leistung überlegen bleibt. Entsprechendes gilt für den Zweikammer-Photoreaktor. In jedem Falle kommt zu der überlegenen Wirtschaftlichkeit des Mehrkammer-Photoreaktors bei der Entkeimung und der photo-oxidativen Wasserreinigung die überlegene Anpassung an veränderliche UV-Durchlässigkeiten gerade auch im Reinstwasserbereich.

Wie in der früheren Anmeldung EP-A-773 beschrieben worden ist, können die vorstehend erwähnten Photoreaktoren ohne weiteres mit anderen Einrichtungen kombiniert werden. So lassen sich die in Fig. 2 bis 4 dargestellten Photoreaktoren unmittelbar mit der in Fig. 10 der vorhergehenden Anmeldung gezeigten Druckausgleichseinrichtung kombinieren, und es ist ebenso unmittelbar zu erkennen, daß solche Photoreaktoren ohne weiteres in ein Rücklaufsystem nach Fig. 13 bis 15 der vorhergehenden Anmeldung eingebaut werden können. Auch kann die Durchströmungsgeschwindigkeit durch die Photoreaktoren, wie bereits früher beschrieben, leicht mittels der Steuereinrichtung nach Fig. 15 der früheren Anmeldung gesteuert werden. Schließlich läßt sich auch die Schichtdicke der die jeweiligen Hüllrohre umgebenden Bestrahlungskammern und der Abstand zwischen den einzelnen Bestrahlungseinheiten bei dem Tankreaktor nach Fig. 2 bis 8 der früheren Anmeldung so wählen, daß die vorstehend angegebenen Optimalbedingungen für annulare Zwei- oder Dreikammer-Photoreaktoren wenigstens annähernd verwirklicht werden.

**Patentansprüche**

1. Verfahren zur Reinigung, bei dem ein fließfähiges Medium zur Einhaltung einer vorbestimmten Mindestdosis ultravioletter Strahlung im Wellenlängenbereich von 240 bis 320 nm, vorzugsweise 260 bis 280 nm, mit einem bestimmten Durchfluß durch einen Durchflußreaktor gefördert wird, der durch nicht mehr als zwei für die ultraviolette Strahlung durchlässige Trennwände senkrecht zur Durchstrahlungsrichtung in nicht mehr als drei in Durchströmungsrichtung hintereinandergeschaltete Bestrahlungskammern jeweils gleichbleibender Schichtdicke unterteilt ist, wobei ein Bruchteil von mindestens 50% der Strahlung, die in die der Strahlungsquelle unmittelbar benachbarte Bestrahlungskammer eindringt, mindestens in die direkt folgende Bestrahlungskammer fällt, und die in den Durchflußreaktor eindringende Strahlung in allen Bestrahlungskammern zusammen höchstens zu 87,5% absorbiert wird, dadurch gekennzeichnet, daß das Medium durch zwei in Durchstrahlungsrichtung und in Durchströmungsrichtung unmittelbar hintereinander angeordnete Bestrahlungskammern gefördert wird, in deren engerer im Bereich von 30,8 bis 47,4 und in denen zusammen im Bereich von 64,5 bis 83,2% der eindringenden Strahlung absorbiert wird.

2. Verfahren nach Anspruch 1, dadurch gekennzeichnet, daß das Medium durch die der Strahlungsquelle unmittelbar benachbarte Bestrahlungskammer mit einer Strömungsgeschwindigkeit oberhalb des Bereiches von 0.1 bis 0.3 m/s gefördert wird.

3. Verfahren nach Anspruch 1 oder 2, dadurch gekennzeichnet, daß das Medium in dem Durchflußreaktor einer in diesen eindringenden Strahlungsleistung von wenigstens 0.5 Watt pro cm effektiver Kammerlänge der UV-Strahlung im wirksamen Wellenlängenbereich ausgesetzt wird.

4. Verfahren nach einem der Ansprüche 1 bis 3, dadurch gekennzeichnet, daß das zu bestrahlende Medium in der der Strahlungsquelle unmittelbar benachbarten Bestrahlungskammer einer UV-Strahlungsdosis von wenigstens 12 Milliwattsekunden pro $cm^2$ ausgesetzt wird.

5. Vorrichtung zur Ausübung des Verfahrens nach einem der Ansprüche 1 bis 4, bestehend aus einer Strahlungsquelle mit mindestens einem Strahler (6, 24), aus einem durch nicht mehr als zwei für die ultraviolette Strahlung durchlässige Trennwände (7, 106, 107, 207, 606) in nicht mehr als drei in Durchströmungsrichtung hintereinandergeschaltete Bestrahlungskammern (8, 9, 109, 110, 111; 209, 211; 609, 611) gleichbleibender Schichtdicke unterteilten Durchflußreaktor (1, 100, 200, 600) mit einer Zuleitung (11, 152, 224, 624) und einer Ableitung (16, 147, 247, 647) für das zu bestrahlende Medium, dessen Bestrahlungskammern (8, 9, 109, 110, 111, 209, 211, 609, 611) in bezug auf die durch die Strahlungsquelle bestimmte Durchstrahlungsrichtung hintereinander angeordnet sind, wobei die in das Medium mindestens in der Bestrahlungskammer (9, 110, 211, 611), die der der Strahlungsquelle unmittelbar benachbarten Bestrahlungskammer (8, 109, 209, 609) direkt folgt, einfallende Strahlung einen Bruchteil von mindestens 50% und mehr der Strahlung beträgt, die in die der Strahlungsquelle unmittelbar benachbarte Bestrahlungskammer (8, 109, 209, 609) eindringt, und die Gesamtschichtdicke aller Bestrahlungskammern nach einer Absorption von nicht mehr als 87,5% der in den Durchflußreaktor (1, 100, 200, 600) eindringenden Strahlung bemessen ist, dadurch gekennzeichnet, daß der Durchflußreaktor (1, 100, 200, 600) zwei in Durchstrahlungsrichtung und in Durchströmungsrichtung unmittelbar hintereinander angeordnete Bestrahlungskammern (8, 9; 109, 110; 110, 111; 209, 211; 609, 611) aufweist, deren engere eine Schichtdicke entsprechend einer Absorption im Bereich von 30,8% bis 47,4% hat und die beide zusammen eine Schichtdicke entsprechend einer Absorption im Bereich von 64,5% bis 83,2% haben.

6. Mehrkammer-Photoreaktor nach Anspruch 5, dadurch gekennzeichnet, daß ein hinsichtlich der Durchflußdosisleistung optimierter Zweikammer-Photoreaktor (1) mit paralleler Durchstrahlung eine nach einer dekadischen Extinktion im Bereich von E = 0.275 bemessene Schichtdicke ($d_1$) der der Strahlungsquelle (6) unmittelbar benachbarten Bestrahlungskammer (8) und eine nach einer dekadischen Extinktion im Bereich von E = 0.710 bemessene Gesamtschichtdicke ($d_1 + d_2$) hat.

7. Mehrkammer-Photoreaktor nach Anspruch 5, dadurch gekennzeichnet, daß ein für Medien einer UV-Durchlässigkeit im Bereich von T (1 cm) 0.2 bis 0.9 (1 cm Küvette; Wellenlänge 254 nm) hinsichtlich der Durchflußdosisleistung optimierter annularer Zweikammer-Photoreaktor (200, 600) mit radial nach

0 014 427

außen gerichteter Durchstrahlung eine nach einer dekadischen Extinktion im Bereich von E = 0.16 bis 0.25 bemessene Schichtdicke ($d_1$) der der Strahlungsquelle (24) unmittelbar benachbarten Bestrahlungskammer (209, 609) und eine nach einer dekadischen Extinktion im Bereich von E = 0.45 bis 0.65 bemessene Gesamtschichtdicke ($d_1 + d_2$) hat.

8. Mehrkammer-Photoreaktor nach Anspruch 7, dadurch gekennzeichnet, daß ein hinsichtlich der Durchflußdosisleistung für ein Medium bestimmter UV-Durchlässigkeit (T (1 cm); 1 cm Küvette; Wellenlänge 254 nm) optimierter Zweikammer-Photoreaktor (200, 600) zur Anpassung an nach niedrigeren Werten der UV-Durchlässigkeit veränderliche Medien eine nach einer dekadischen Extinktion im Bereich von E = 0.16 bemessene Schichtdicke ($d_1$) der der Strahlungsquelle (24) unmittelbar benachbarten Bestrahlungskammer (209, 609) und eine nach einer dekadischen Extinktion im Bereich von E = 0.47 bemessene Gesamtschichtdicke ($d_1 + d_2$) hat.

9. Mehrkammer-Photoreaktor nach Anspruch 7, dadurch gekennzeichnet, daß ein hinsichtlich der Durchflußdosisleistung für ein Medium bestimmter UV-Durchlässigkeit (T (1 cm); 1 cm Küvette; Wellenlänge 254 nm) optimierter Zweikammer-Photoreaktor (200, 600) zur Anpassung an nach höheren Werten der UV-Durchlässigkeit veränderliche Medien eine nach einer dekadischen Extinktion im Bereich von E = 0.23 bemessene Schichtdicke ($d_1$) der der Strahlungsquelle (24) unmittelbar benachbarten Bestrahlungskammer (209, 609) und eine nach einer dekadischen Extinktion im Bereich von E = 0.60 bemessene Gesamtschichtdicke ($d_1 + d_2$) hat.

10. Zweikammer-Photoreaktor nach Anspruch 7, dadurch gekennzeichnet, daß ein hinsichtlich der Durchflußdosisleistung für ein Medium einer UV-Durchlässigkeit von T (1 cm) 0.75 (1 cm Küvette; Wellenlänge 254 nm) optimierter annularer Zweikammer-Photoreaktor (600) eine der Strahlungsquelle unmittelbar benachbarte Bestrahlungskammer (609) mit einer Schichtdicke ($d_1$) von 1.5 cm und eine Gesamtschichtdicke ($d_1 + d_2$) von 3.6 cm hat.

11. Zweikammer-Photoreaktor nach Anspruch 10, dadurch gekennzeichnet, daß die der Strahlungsquelle unmittelbar benachbarte Bestrahlungskammer (609) von einem die Strahlungsquelle nach Art einer Tauchlampe aufnehmenden, einseitig geschlossenen Hüllrohr (605) und einem das Hüllrohr (605) koaxial umgebenden, über den Bereich der Bogenlänge des Strahlers aus einem für die UV-Strahlung durchlässigen Material bestehenden Trennrohr (606) gebildet ist, daß das Hüllrohr (605) und das Trennrohr (606) gemeinsam abdichtend in ein koaxial dazu angeordnetes, einseitig offenes Gefäß (602) aus für die UV-Strahlung undurchlässigem Material, das mindestens zwei seitliche Anschlüsse (647) und eine Beobachtungseinrichtung (620) aufweist und mit dem Trennrohr (606) zusammen die zweite Bestrahlungskammer (611) bildet, eingesetzt sind.

12. Zweikammer-Photoreaktor nach Anspruch 11, dadurch gekennzeichnet, daß das Gefäß (602) aus Quarzgut besteht, das an seinem offenen Ende eine plan geschliffene Stirnfläche (612) aufweist, und daß das Trennrohr (606) einen mit Planschliff versehenen, an die Stirnfläche (612) des Gefäßes (602) angepaßten Ringflansch (616) und an seinem außerhalb des Gefäßes (602) befindlichen Ende einen mit einem entsprechend ausgebildeten Ende des Hüllrohres (605) abdichtend verbundenen Flansch (615) trägt.

13. Mehrkammer-Photoreaktor nach Anspruch 5, dadurch gekennzeichnet, daß ein annularer Dreikammer-Photoreaktor mit radial nach außen gerichteter Durchstrahlung aus einem hinsichtlich der Durchflußdosisleistung optimierten Zweikammer-Photoreaktor und einer damit unter Optimierung der Durchflußdosisleistung kombinierten weiteren Bestrahlungskammer besteht.

14. Dreikammer-Photoreaktor nach Anspruch 13, dadurch gekennzeichnet, daß ein für Medien einer UV-Durchlässigkeit im Bereich von T (1 cm) 0.2 bis 0.9 (1 cm Küvette; Wellenlänge 254 nm) hinsichtlich der Durchflußdosisleistung optimierter Dreikammer-Photoreaktor (100) eine nach einer dekadischen Extinktion im Bereich von E = 0.14 bis 0.20 bemessene Schichtdicke ($d_1$) der der Strahlungsquelle (24) unmittelbar benachbarten Bestrahlungskammer (109) und eine nach einer dekadischen Extinktion im Bereich von E = 0.60 bis 0.80 bemessene Gesamtschichtdicke ($d_1 + d_2 + d_3$) hat.

15. Dreikammer-Photoreaktor nach Anspruch 13, dadurch gekennzeichnet, daß ein hinsichtlich der Durchflußdosisleistung für ein Medium bestimmter UV-Durchlässigkeit (T (1 cm); 1 cm Küvette; Wellenlänge 254 nm) optimierter Dreikammer-Photoreaktor (100) zur Anpassung an nach niedrigeren Werten der UV-Durchlässigkeit veränderliche Medien eine nach einer dekadischen Extinktion im Bereich von E = 0.14 bemessene Schichtdicke ($d_1$) der der Strahlungsquelle (24) unmittelbar benachbarten Bestrahlungskammer (109) und eine nach einer dekadischen Extinktion im Bereich von E = 0.61 bemessene Gesamtschichtdicke ($d_1 + d_2 + d_3$) hat.

16. Dreikammer-Photoreaktor nach Anspruch 13, dadurch gekennzeichnet, daß ein hinsichtlich der Durchflußdosisleistung für ein Medium bestimmter UV-Durchlässigkeit (T (1 cm); 1 cm Küvette; Wellenlänge 254 nm) optimierter Dreikammer-Photoreaktor (100) zur Anpassung an nach höheren Werten der UV-Durchlässigkeit veränderliche Medien eine nach einer dekadischen Extinktion im Bereich von E = 0.19 bemessene Schichtdicke ($d_1$) der der Strahlungsquelle (24) unmittelbar benachbarten Bestrahlungskammer (109) und eine nach einer dekadischen Extinktion im Bereich von E = 0.79 bemessene Gesamtschichtdicke ($d_1 + d_2 + d_3$) hat.

17. Dreikammer-Photoreaktor nach Anspruch 5, dadurch gekennzeichnet, daß ein hinsichtlich der Durchflußdosisleistung optimierter und an Medien einer UV-Durchlässigkeit im Bereich von T (1 cm) 0.1 bis 0.99 (1 cm Küvette; Wellenlänge 254 nm) angepaßter Dreikammer-Photoreaktor (100) aus einem der Strahlungsquelle (24) unmittelbar benachbarten, hinsichtlich der Durchflußdosisleistung für Medien einer UV-Durchlässigkeit im Bereich von T (1 cm) 0.4 bis 0.6 optimierten Zweikammer-Photoreaktor und einer weiteren, diesen umgebenden Bestrahlungskammer (111) einer Schichtdicke (d3) = 3.5 cm gebildet ist.

18. Mehrkammer-Photoreaktor nach einem der Ansprüche 5 bis 17, dadurch gekennzeichnet, daß die Strahlungsquelle ein Strahler hoher Strahlungsleistung, vorzugsweise von mehr als 0.5 w/cm effektiver Kammerlänge, der UV-Strahlung im wirksamen Wellenlängenbereich ist.

19. Verwendung eines für ein Medium einer vorgegebenen Transmission T (1 cm) (1 cm Küvette; Wellenlänge 254 nm) optimierten Zweikammer-Photoreaktors nach einem der Ansprüche 5 bis 18 zur Reinigung von Medien starkveränderlicher Zusammensetzung oder unterschiedlicher Wasserqualitäten.

20. Verwendung eines für ein Medium einer Transmission von T (1 cm) = 0.7 (1 cm Küvette; Wellenlänge 254 nm) optimierten Zweikammer-Photoreaktors nach Anspruch 6 zur Reinigung von Medien mit einer Transmission im Bereich von T (1 cm) = 0.6 bis T (1 cm) = 0.9.

21. Verwendung eines für ein Medium einer Transmission von T (1 cm) = 0.5 (1 cm Küvette; Wellenlänge 254 nm) optimierten Zweikammer-Photoreaktors nach Anspruch 6 zur Reinigung von Medien mit einer Transmission im Bereich von T (1 cm) = 0.75.

22. Verwendung von Mehrkammer-Photoreaktoren nach einem der Ansprüche 5 bis 18, die hinsichtlich der Durchflußdosisleistung für Medien einer UV-Durchlässigkeit im Bereich von T (1 cm) 0.65, vornehmlich 0.75, (1 cm Küvette; Wellenlänge 254 nm) optimiert sind, zur Reinigung, insbesondere zur Entkeimung und Desinfektion von Medien einer UV-Durchlässigkeit im Bereich von T (1 cm) 0.8 und darüber.

23. Verwendung von Mehrkammer-Photoreaktoren nach einem der Ansprüche 5 bis 18, die hinsichtlich der Durchflußdosisleistung für Medien einer UV-Durchlässigkeit im Bereich von T (1 cm) 0.9 (1 cm Küvette; Wellenlänge 254 nm) optimiert sind, zur Reinigung, insbesondere zur Entkeimung und Desinfektion von Wasser mit einer UV-Durchlässigkeit im Bereich von T (1 cm) oberhalb 0.9.

## Claims

1. Method of purification in which a medium capable of flowing is passed through a throughflow reactor at a defined rate of flow for maintaining a predetermined minimum dose of ultraviolet radiation of a wavelength in the range of 240 to 320 nm, preferably 260 to 280 nm, said flow reactor being subdivided perpendicularly to the direction of irradiation by not more than two partitions, which are transparent for the ultraviolet radiation, into not more than three irradiation chambers which are series-connected with respect to the throughflow direction and each of which has a constant thickness, wherein a fraction of at least 50% of the radiation entering the irradiation chamber immediately adjacent to the radiation source falls into at least the immediately following irradiation chamber and wherein a maximum of 87.5% of the radiation entering the flow reactor is conjointly absorbed in all irradiation chambers, characterised in that the medium is passed through two irradiation chambers arranged immediately one after the other with respect to the direction of irradiation and with respect to the throughflow direction, in the range of 30.8 to 47.4% of the entering radiation being absorbed in the narrower one of the two irradiation chambers and in the range of 64.5 to 83.2% of the entering radiation being absorbed conjointly in the two irradiation chambers.

2. Method according to claim 1, characterised in that the medium is passed through the irradiation chamber immediately adjacent the radiation source at a flow rate above the range of 0.1 to 0.3 m/s.

3. Method according to claim 1 or claim 2, characterised in that the medium is exposed in the throughflow reactor to a radiation intensity which enters the throughflow reactor and amounts to at least 0.5 Watt per cm of the effective chamber length, of the UV radiation in the effective wavelength range.

4. Method according to any one of claims 1 to 3, characterised in that the medium to be irradiated is exposed to a UV radiation dose of at least 12 milliWattseconds per $cm^2$ in the irradiation chamber immediately adjacent to the radiation source.

5. Apparatus for carrying out the method according to any one of claims 1 to 4, comprising a radiation source containing at least one radiation emitter (6, 24), a throughflow reactor (1, 100, 200, 600) subdivided by not more than two partitions (7, 106, 107, 207, 606) which are transparent for the ultraviolet radiation, into not more than three irradiation chambers (8, 9, 109, 110, 111, 209, 211, 609, 611) which are series-connected with respect to the throughflow direction and which have a constant thickness, and comprising an infeed (11, 152, 224, 624) and an outfeed (16, 147, 247, 647) for the medium to be irradiated, the irradiation chambers (8, 9, 109, 110, 111, 209, 211, 609, 611) of the throughflow reactor being series-arranged with respect to the direction of irradiation as defined by the radiation source, wherein the radiation entering the medium at least in the irradiation chamber (9, 110, 211, 611) immediately following the irradiation chamber (8, 109, 209, 609) immediately adjacent to the radiation source amounts to a fraction of at least 50% and more of the radiation entering the irradiation chamber (8, 109, 209, 609) immediately adjacent to the radiation source, and wherein the total thickness of all irradiation chambers is measured by an absorption of not more than 87.5% of the radiation entering the throughflow reactor (1, 100, 200, 600), characterised in that the throughflow reactor (1, 100, 200, 600) comprises two irradiation chambers (8, 9; 109, 110, 111; 209, 211; 609, 611) which are arranged immediately one after the other with respect to the direction of irradiation und with respect to the throughflow direction, the narrower one of which has a thickness corresponding to an absorption in the range of 30.8% to 47.4% and both of which conjointly have a thickness corresponding to an absorption in the range of 64.5% to 83.2%.

6. Multichamber photoreactor according to claim 5, characterised in that a two-chamber photoreactor (1) which is traversed by parallel radiation and which is optimized with respect to the flow-dose rate, has a thickness $(d_1)$ of the irradiation chamber (8) immediately adjacent to the radiation source (6) and which thickness is measured by a decadic extinction in the range of E = 0.275, and has a total thickness $(d_1 + d_2)$ measured by a decadic extinction in the range of E = 0.710.

7. Multichamber photoreactor according to claim 5, characterised in that an annular two-chamber photoreactor (200, 600), which is traversed by radially outwardly directed radiation and which is optimized with respect to the flow-dose rate for media having a UV transmission (1 cm cuvette; wavelength 254 nm) in the range of T (1 cm) 0.2 to 0.9, has a thickness $(d_1)$ of the irradiation chamber (209, 609) immediately adjacent to the radiation source (24) and which thickness is measured by a decadic extinction in the range of E = 0.16 to 0.25, and has a total thickness $(d_1 + d_2)$ measured by a decadic extinction in the range of E = 0.45 to 0.65.

8. Multichamber photoreactor according to claim 7, characterised in that a two-chamber photoreactor (200, 600) which is optimized with respect to the flow-dose rate for a medium having a predetermined UV transmission (T (1 cm); 1 cm cuvette; wavelength 254 nm), for adaptation to media having a UV transmission variable to lower values of the UV transmission, has a thickness $(d_1)$ of the irradiation chamber (209, 609) immediately adjacent to the radiation source (24) and which thickness is measured by a decadic extinction in the range of E = 0.16, and has a total thickness $(d_1 + d_2)$ measured by a decadic extinction in the range of E = 0.47.

9. Multichamber photoreactor according to claim 7, characterised in that a two-chamber photoreactor (200, 600) which is optimized with respect to the flow-dose rate for a medium having a predetermined UV transmission (T (1 cm); 1 cm cuvette; wavelength 254 nm), for adaptation to media having UV transmissions variable to higher values of the UV transmission, has a thickness $(d_1)$ of the irradiation chamber (209, 609) immediately adjacent to the radiation source (24) and which thickness is measured by a decadic extinction in the range of E = 0.23, and has a total thickness $(d_1 + d_2)$ measured by a decadic extinction in the range of E = 0.60.

10. Two-chamber photoreactor according to claim 7, characterised in that an annular two-chamber photoreactor (600) which is optimized with respect to the flow-dose rate for a medium having a UV transmission of T (1 cm) 0.75 (T (1 cm); 1 cm cuvette; wavelength 254 nm), has an irradiation chamber (609) immediately adjacent to the radiation source and which has a thickness $(d_1)$ of 1.5 cm, and has a total thickness $(d_1 + d_2)$ of 3.6 cm.

11. Two-chamber photoreactor according to claim 10, characterised in that the irradiation chamber (609) immediately adjacent to the radiation source is formed by a unilaterally closed envelope tube (605) which accomodates the radiation source in the manner of an immersion-type lamp, and by a partition tube (606) which coaxially surrounds the envelope tube (605) and consists of a material transparent for the UV radiation over the range of the arc length of the radiation emitter, that the envelope tube (605) and the partition tube (606) are conjointly and sealingly inserted into a unilaterally open vessel (602) which is coaxially arranged thereto, which is made of a material opaque for the UV radiation, which comprises at least two lateral connections (647) and an observation device (620) and which forms the second irradiation chamber (611) conjointly with said partition tube (606).

12. Two-chamber photoreactor according to claim 11, characterised in that the vessel (602) is made of vitreous silica and comprises at its open end a planar, ground end face (612) and that the partition tube (606) carries an annular flange (616), which is surface ground and matched to the end face (612) of the vessel (602), and a flange (615) which is located at the end outside of the vessel (602) and which is sealingly connected to a correspondingly designed end of the envelope tube (605).

13. Multichamber photoreactor according to claim 5, characterised in that an annular three-chamber photoreactor which is traversed by radially outwardly directed irradiation, comprises a two-chamber photoreactor which is optimized with respect to the flow-dose rate, and a further irradiation chamber combined therewith with an optimization of the flow-dose rate.

14. Three-chamber photoreactor according to claim 13, characterised in that a three-chamber photoreactor (100) which is optimized with respect to the flow-dose rate for media having a UV transmission in the range of T (1 cm) 0.2 to 0.9 (1 cm cuvette; wavelength 254 nm), has an irradiation chamber (109) immediately adjacent to the radiation source and having a thickness $(d_1)$ measured by a decadic extinction in the range of E = 0.14 to 0.20, and has a total thickness $(d_1 + d_2 + d_3)$ measured by a decadic extinction in the range of E = 0.60 to 0.80.

15. Three-chamber photoreactor according to claim 13, characterised in that a three-chamber photoreactor (100), which is optimized with respect to the flow-dose rate for a medium having a predetermined UV transmission (T (1 cm); 1 cm cuvette; wavelength 254 nm), for adaptation to media having UV transmissions variable to lower values of the UV transmission, has a thickness ($d_1$) of the irradiation chamber (109) immediately adjacent to the radiation source and which thickness is measured by a decadic extinction in the range of E = 0.14, and has a total thickness ($d_1 + d_2 + d_3$) measured by a decadic extinction in the range of E = 0.61.

16. Three-chamber photoreactor according to claim 13, characterised in that a three-chamber photoreactor (100), which is optimized with respect to the flow-dose rate for a medium having a predetermined UV transmission (T (1 cm); 1 cm cuvette; wavelength 254 nm), for adaption to media having UV transmissions variable to higher values of the UV transmission, has a thickness ($d_1$) of the irradiation chamber (109) immediately adjacent to the radiation source (24) and which thickness is measured by a decadic extinction in the range of E = 0.19, and has a total thickness ($d_1 + d_2 + d_3$) measured by a decadic extinction in the range of E = 0.79.

17. Three-chamber photoreactor according to claim 5, characterised in that a three-chamber photoreactor (100), which is optimized with respect to the flow-dose rate and which is adapted to media having a UV transmission in the range of T (1 cm) 0.1 to 0.99 (1 cm cuvette; wavelength 254 nm), is formed by a two-chamber photoreactor immediately adjacent to the radiation source (24) and optimized with respect to the flow-dose rate for media having a UV transmission in the range of T (1 cm) 0.4 to 0.6, and a further irradiation chamber (111) surrounding the two-chamber photoreactor and having a thickness ($d_3$) = 3.5 cm.

18. Multichamber photoreactor according to any one of claims 5 to 17, characterised in that the radiation source constitutes a high-power radiation emitter for the UV radiation in the effective wavelength range, preferably of an effective chamber length greater than 0.5 W per cm.

19. Method of using a two-chamber photoreactor according to any one of claims 5 to 18, which two-chamber photoreactor is optimized for a medium of a predetermined transmission T (1 cm) (1 cm cuvette; wavelength 254 nm) for purifying media of strongly varying composition or variable water qualities.

20. Method of using a two-chamber photoreactor according to claim 6 and optimized for a medium having a transmission of T (1 cm) = 0.7 (1 cm cuvette; wavelength 254 nm) for purifying media having a transmission in the range of T (1 cm) = 0.6 to T (1 cm) = 0.9.

21. Method of using a two-chamber photoreactor according to claim 6 and optimized for a medium having a transmission of T (1 cm) = 0.5 (1 cm cuvette; wavelength 254 nm) for purifying media having a transmission in the range of T (1 cm) = 0.75.

22. Method of using multichamber photoreactors according to any one of claims 5 to 18 which are optimized with respect to the flow-dose rate for media having a UV transmission in the range of T (1 cm) 0.65, preferably 0.75, (1 cm cuvette; wavelength 254 nm) for purification, particularly sterilisation und disinfection of media having a UV transmission in the range of T (1 cm) 0.8 and thereabove.

23. Method of using multichamber photoreactors according to any one of claims 5 to 18 which are optimized with respect to the flow-dose rate for media having a UV transmission in the range of T (1 cm) 0.9 (1 cm cuvette; wavelength 254 nm), for purification, particularly sterilisation and disinfection of water having a UV transmission in the range of T (1 cm) above 0.9.

## Revendications

1. Procédé de purification dans lequel, pour maintenir une dose minimale prédéterminée de rayonnement ultraviolet dans la gamme d'ondes de 240 à 329 nm, de préférence 260 à 280 nm, un milieu fluide est refoulé, à un débit déterminé, à travers un réacteur à écoulement subdivisé par au maximum deux cloisons laissant passer les rayons ultraviolets, perpendiculairement à la direction d'irradiation, en au maximum trois chambres d'irradiation disposées en série relativement à la direction d'écoulement et d'une épaisseur de couche constante, une partie d'au moins 50% du rayonnement qui pénètre dans la chambre d'irradiation immédiatement voisine de la source de rayons, tombant dans la chambre d'irradiation immédiatement voisine, et le rayonnement qui pénètre dans le réacteur à écoulement étant absorbé dans toutes les chambres d'irradiation ensemble, à maximalement 87,5%, procédé caractérisé par le fait que le milieu est refoulé à travers deux chambres d'irradiation disposées directement en série relativement à la direction d'irradiation et d'écoulement, dans la plus étroites, des chambres d'irradiation, le rayonnement pénétrant étant absorbé de l'ordre de 30,8 à 47,4% et dans les chambres ensemble le rayonnement pénétrant étant absorbé de l'ordre de 64,5 à 83,2%.

2. Procédé selon la revendication 1, caractérisé par le fait que le milieu est refoulé à travers la chambre d'irradiation immédiatement voisine de la source de rayons, avec une vitesse d'écoulement supérieure à une vitesse de l'ordre 0,1 à 0,3 m/sec.

3. Procédé selon la revendication 1 ou 2, caractérisé par le fait que le milieu est exposé, dans le réacteur à écoulement, à une puissance de rayonnement pénétrant dans celui-ci d'au moins 0,5 watt per cm de longueur de chambre effective des rayons ultraviolets dans la gamme d'ondes active.

4. Procédé selon l'une quelconque des revendications 1 à 3, caractérisé par le fait que le milieu à irradier est exposé, dans la chambre d'irradiation immédiatement voisine de la source de rayons, à une dose de rayons ultraviolets d'au moins 12 milliwatt-secondes per $cm^2$.

5. Appareil pour la mise en oeuvre du procédé selon l'une quelconque des revendications 1 à 4, comprenant une source de rayons qui comporte au moins un émetteur (6, 24), un réacteur à écoulement (1, 110, 200, 600) qui est subdivisé par maximalement deux cloisins (7, 106, 107, 207, 606) laissant passer les rayons ultraviolets, en maximalement trois chambres d'irradiation (8, 9, 109, 110, 111, 209, 211, 609, 611) disposées en série relativement à la direction d'écoulement et d'une épaisseur de couche constante, et quel réacteur à écoulement est muni d'une conduite d'amenée (11, 152, 224, 624) et d'une conduite d'évacuation (16, 147, 247, 647) pour le milieu à irradier, et dont les chambres d'irradiation (8, 9, 109, 110, 111, 209, 211, 609, 611) sont disposées en série relativement à la direction d'irradiation définie par le source de rayons, le rayonnement qui pénètre dans le milieu au moins dans la chambre d'irradiation (9, 110, 211, 611) qui fait directement suite à la chambre d'irradiation (8, 109, 209, 609) immédiatement voisine de la source de rayons, s'élevant à une fraction d'au moins 50% et plus du rayonnement qui pénètre dans la chambre d'irradiation (8, 109, 209, 609) immédiatement voisine de la source de rayons, et l'épaisseur totale de couche de toutes les chambres d'irradiation étant dimensionnée partir d'une absorption de maximalement 87,5% du rayonnement qui pénètre dans le réacteur à écoulement (1, 100, 200, 600), appareil caractérisé par le fait que le réacteur d'écoulement (1, 100, 200, 600) comprend deux chambre d'irradiation (8, 9; 109, 110; 110, 111; 209, 211; 609, 611) disposées directement en série relativement à la direction d'irradiation et d'écoulement, et dont la plus étroite a une épaisseur de couche correspondant à une absorption de l'ordre de 30,8% à 47,4% et qui ensemble ont une épaisseur de couche correspondant à une absorption de l'ordre de 64,5% à 83,2%.

6. Photoréacteur à chambres multiples selon la revendication 5, caractérisé par le fait qu'un photoréacteur à deux chambres (1) optimisé relativement à la puissance de dose d'écoulement et avec une irradiation parallèle, a une épaisseur de couche $(d_1)$ de la chambre d'irradiation (8) immédiatement voisine de la source de rayons (6), épaisser dimensionée selon une extinction décimal dans le domaine de $E = 0,275$, et une épaisseur totale de couche $(d_1 + d_2)$ dimensionée selon une extinction décimal dans le domaine de $E = 0,710$.

7. Photoréacteur à chambres multiples selon la revendication 5, caractérisé par le fait qu'un photoréacteur annulaire à deux chambres (200, 600) optimise, en ce qui concerne la puissance de dose d'écoulement, pour des milieux d'une transmission pour des rayons ultraviolets dans le domaine de T (1 cm) 0,2 à 0,9 (1 cm cuvette, longueur d'onde 254 nm) et avec une irradiation dirigée radialement vers l'extérieur, a une épaisseur de couche $(d_1)$ de la chambre d'irradiation (209, 609) immédiatement voisine de la source de rayons, épaisseur dimensionée selon une extinction décimal dans le domaine de $E = 0,16$ à 0,25, et une épaisseur totale de couche $(d_1 + d_2)$ dimensionée selon une extinction décimal dans le domaine de $E = 0,45$ à 0,65.

8. Photoréacteur à chambres multiples selon la revendication 7, caractérisé par le fait qu'un photoréacteur à deux chambres (200, 600) optimisé en ce qui concerne la puissance de dose d'écoulement pour un milieu d'une transmission déterminée pour des rayons ultraviolets (T (1 cm cuvette; longueur d'onde 254 nm), possède, pour adaptation à des milieux variables selon des valeurs inférieurs de la transmission pour des rayons ultraviolets, und épaisseur de couche $(d_1)$ de la chambre d'irradiation (209, 609) immédiatement voisine de la source de rayons (24), épaisseur dimensionnée selon une extinction décimal dans le domaine de $E = 0,16$, et une épaisseur totale de couche $(d_1 + d_2)$ dimensionnée selon une extinction décimal dans le domaine de $E = 0,47$.

9. Photoréacteur à chambres multiples selon la revendication 7, caractérisé par le fait qu'un photoréacteur à deux chambres (200, 600) optimisé en ce qui concerne la puissance de dose d'écoulement pour un milieu d'une transmission déterminée pour des rayons ultraviolets (T (1 cm); 1 cm cuvette; longueur d'onde 254 nm), possède, pour l'adaption à des milieux variables selon des valeurs supérieures de la transmission pour des rayons ultraviolets, une épaisseur de couche $(d_1)$ de la chambre d'irradiation (209, 609) immédiatement voisine de la source de rayons (24), épaisseur dimensionée selon l'extinction décimal dans le domaine de $E = 0,23$, et une épaisseur totale de couche $(d_1 + d_2)$ dimensionnée selon une extinction décimal dans le domaine de $E = 0,60$.

10. Photoréacteur à deux chambres selon la revendication 7, caractérisé par le fait qu'un photoréacteur annulaire à deux chambres (600) optimisé en ce qui concerne la puissance de dose d'écoulement, pour un milieu d'une transmission pour des rayons ultraviolets de T (1 cm) 0,75 (1 cm cuvette, longueur d'onde 254 nm) comprend une chambre d'irradiation (609) immédiatement voisine de la source de rayons et ayant une épaisseur de couche $(d_1)$ de 1,5 cm et une épaisseur totale de couche $(d_1 + d_2)$ de 3,6 cm.

11. Photoréacteur à deux chambres selon la revendication 10 caractérisé par le fait que la chambre d'irradiation (609) immédiatement voisine de la source de rayons est formée par un tube d'enveloppe (605) fermée d'un côté et recevant la source de rayons à l'instar d'une lampe à immersion, et par un tube séparateur (606) entourant le tube d'enveloppe coaxialement, et consistant, dans le domaine de la longueur de l'arc de l'émetteur, d'une matière laissant passer les rayons ultraviolets, et par le fait que le tube d'enveloppe (605) et le tube séparateur (606) sont introduits ensemble, d'une manière étanchante, dans un récipient (602) ouvert d'un côté, disposé coaxialement à ceux-ci et consistant d'une matière ne laissant pas passer les rayons ultraviolets, récipient possédant au moins deux connexions (647) latérales et un dispositif d'observation (620) et formant avec le tube séparateur (606) la deuxième chambre d'irradiation (611).

12. Photoréacteur à deux chambres selon la revendication 11, caractérisé par le fait que le récipient

23

(602) consiste de silice fondue qui présente, à son bout ouvert, une surface frontal (612) à réctification plane et que le tube séparateur (606) porte une flasque annulaire (616) prévue d'une rectification plane et adaptée à la surface frontal (612) du récipient (602), et, au bout se trouvant à l'extérieur de récipient (602), une flasque (615) reliée d'une manière étanchante à un bout de tube d'enveloppe (605) formée d'une manière correspondante.

13. Photoréacteur à chambres multiples selon la revendication 5, caractérisé par le fait que photoréacteur annulaire à trois chambres avec une irradiation dirigée radialement vers l'extérieur consiste d'un photoréacteur à deux chambres optimisé en ce qui concerne la puissance de dose d'écoulement, et d'une autre chambre d'irradiation combinée avec celui-ci en optimisant la puissance de dose d'écoulement.

14. Photoréacteur à trois chambres selon la revendication 13, caractérisé par le fait qu'un photoréacteur à trois chambres (100) optimisé, en ce qui concerne la puissance de dose d'écoulement, pour des milieux d'une transmission pour des rayons ultraviolets (T (1 cm); 1 cm cuvette; longueur d'onde 254 nm) dans le domaine de T (1 cm) 0,2 à 0,9 possède, une épaisseur de couche $(d_1)$ de la chambre d'irradiation (109) immédiatement voisine de la source de rayons (24), épaisseur dimensionnée selon une extinction décimal dans le domaine de $E = 0,14$ à 0,20, et une épaisseur total de couche $(d_1 + d_i + d_3)$ dimensionée selon une extinction décimal dans le domaine de $E = 0,60$ à 0,80.

15. Photoréacteur à trois chambres selon la revendication 13, caractérisé par le fait qu'un photoréacteur à trois chambres (100) optimisé, en ce qui concerne la puissance de dose d'écoulement, pour un milieu d'une transmission déterminée pour des rayons ultraviolets (T (1cm); 1 cm cuvette; longueur d'onde 254 nm) possède, pour l'adaption à des milieux variables selon des valeurs inférieures de la transmission pour des rayons ultraviolets, une épaisseur de couche $(d_1)$ de la chambre d'irradiation (109) immédiatement voisine de la source de rayons (24), épaisseur dimensionnée selon une extinction décimal dans le domaine de $E = 0,14$, et une épaisseur totale de couche $(d_1 + d_2 + d_3)$ dimensionnée selon une extinction décimal dans le domaine de $E = 0,61$.

16. Photoréacteur à trois chambres selon la revendication 13, caractérisé par le fait qu'un photoréacteur à trois chambres (100) optimisé, en ce qui concerne la puissance de dose d'écoulement, pour un milieu d'une transmission déterminée pour des rayons ultraviolets (T (1 cm); 1 cm cuvette; longueur donde 254 nm) possède, pour l'adaption à des milieux variables selon des valeurs supérieures de la transmission pour des rayons ultraviolets, une épaisseur de couche $(d_1)$ de la chambre d'irradiation (109) immédiatement voisine de la source de rayons (24), épaisseur dimensionnée selon une extinction décimal dans le domaine de $E = 0,19$ et une épaisseur totale de couche $(d_1 + d_2 + d_3)$ dimensionnée selon une extinction décimal dans le domaine de $E = 0,79$.

17. Photoréacteur à trois chambres selon la revendication 5, caractérisé par le fait qu'un photoréacteur à trois chambres (100) optimisé, en ce qui concerne puissance de dose d'écoulement, et adaptée à des milieux d'une transmission pour des rayons ultraviolets dans le domaine T (1 cm) 0,1 à 0,99 (1 cm cuvette; longueur d'onde 254 nm), est formé d'un photoréacteur à deux chambres immédiatement voisine de la source de rayons (24) et optimisé, en ce qui concerne la puissance de dose d'écoulement, pour des milieux d'une transmission pour des rayons ultraviolets dans le domaine de T (1 cm) 0,4 à 0,6 et d'une autre chambre d'irradiation (111) entourant celui-ci, d'une épaisseur de couche $(d_3) = 3,5$ cm.

18. Photoréacteur à chambres multiples selon l'une quelconque des revendications 5 à 17, caractérisé par le fait que la source de rayons est un émetteur d'une puissance de rayonnement élevée, de préférence de plus de 0,5 w/cm de longueur de chambre effective, des rayons ultraviolets dans la gamme d'ondes active.

19. Procédé d'emploi d'un photoréacteur à deux chambres optimisé pour un milieu d'une transmission déterminée T (1 cm) (1 cm cuvette, longueur d'onde 254 nm), selon l'une quelconque des revendications 5 à 18, pour la purification de milieux d'une composition d'une grande variabilité ou de qualités d'eau différentes.

20. Procédé d'emploi d'un photoréacteur à deux chambres optimisé pour un milieu d'une transmission de T (1 cm) = 0,7 (1 cm cuvette, longueur d'onde 254 nm) selon la revendication 6, pour la purification de milieux avec une transmission de l'ordre de T (1 cm) = 0,6 à T (1 cm) = 0,9.

21. Procédé d'emploi d'un photoréacteur à deux chambres optimisé pour un milieu d'une transmission de T (1 cm) = 0,5 (1 cm cuvette, longueur d'onde 254 nm) selon la revendication 6, pour la purification de milieux avec une transmission de l'ordre de T (1 cm) = 0,75.

22. Procédé d'emploi de photoréacteurs à chambres multiples selon l'une quelconques des revendications 5 à 18, photoréacteurs optimisés en ce qui concerne la puissance de dose d'écoulement, pour des milieux d'une transmission pour des rayons ultraviolets dans le domaine de T (1 cm) 0,65, de préférence 0,75, (1 cm cuvette; longueur d'onde 254 nm) pour la purification et particulièrement pour la stérilisation et la désinfection de milieux d'une transmission de rayons ultraviolets de l'ordre de T (1 cm) 0,8 et plus.

23. Procédé d'emploi de photoréacteurs à chambres multiples selon l'une quelconque des revendications 5 à 18, photoréacteurs optimisés en ce qui concerne la puissance de dose d'écoulement, pour des milieux d'une transmission pour des rayons ultraviolets dans le domaine de T (1 cm) 0,9 (1 cm cuvette; longueur d'onde 254 nm) pour la purification et particulièrement pour la stérilisation et la désinfection de milieux d'une transmission de rayons ultraviolets de l'ordre de T (1 cm) supérieure à 0,9.

Fig.1

0 014 427

Fig. 2

Fig. 3

Fig.4